# EUROPEAN PATENT SPECIFICATION

(11) **EP 1 397 501 B1**
(45) Date of publication and mention of the grant of the patent: **23.07.2008**
(21) Application number: 01967078.5
(22) Date of filing: 13.09.2001
(51) Int. Cl.: C12P 7/62

(54) **METHOD OF PRODUCTION OF BIODEGRADABLE LACTIC ACID POLYMERS**
VERFAHREN ZUR HERSTELLUNG VON BIOLOGISCH ABBAUBAREN MILCHSÄUREPOLYMEREN
PROCEDE DE PRODUCTION DE POLYMERES D'ACIDE LACTIQUE BIODEGRADABLES

(30) Priority: 23.03.2001 EE 200100181
(43) Date of publication of application: 17.03.2004
(73) Proprietor: Nordbiochem OÜ, 63303 Pölva (EE)
(72) Inventor: KOLBAKOV, Vambola, EE 63202 Polva vald (EE); NURK, Allan, EE 50105 Tartu (EE); SIMISKER, Jaan, deceased (EE)
(74) Representative: Sarap, Margus
(86) International application number: PCT/EE2001/000007
(87) International publication number: WO 2002/077252

(56) References cited:
- US-A- 5 503 750
- DATABASE WPI Section Ch, Week 200110 Derwent Publications Ltd., London, GB; Class A41, AN 2001-083798 XP002184796 & JP 2000 245491 A (SHIRAI Y), 12 September 2000 (2000-09-12)

### Sequence Listing

<110> MEDEXGEN INC
   CHUNG, Yong Hoon
   CHO, Hoon Sik
   PARK, Hong Gyu
   YI, Ki Wan
<120> PHARMACEUTICAL COMPOSITION FOR TREATMENT OF IMMUNOLOGICAL DISORDERS
<130> P052MED02PCT
<150> KR10-2004-0010835
   <151> 2004-02-18
<160> 26
<170> KopatentIn 1.71
<210> 1
   <211> 31
   <212> DNA
   <213> Artificial Sequence
<220>
   <223> primer, oligo-LAG3-F-EcoRI
<400> 1
   ggaattcatg tgggaggctc agttcctggg c 31
<210> 2
   <211> 28
   <212> DNA
   <213> Artificial Sequence
<220>
   <223> primer, oligo-LAG3-R-5P
<400> 2
   agtgaggtta tacatgatgg agacgttg 28
<210> 3
   <211> 21
   <212> DNA
   <213> Artificial Sequence
<220>
   <223> primer, oligo-LAG3-F-5P
<400> 3
   ctccagccag gggctgaggt c 21
<210> 4
   <211> 30
   <212> DNA
   <213> Artificial Sequence
<220>
   <223> primer, oligo-LAG3-R-SpeI
<400> 4
   gactagttgg gggctccaga cccagaacag 30
<210> 5
   <211> 28
   <212> DNA
   <213> Artificial Sequence
<220>
   <223> primer, hIgG-F-SpeI
<400> 5
   gagtagtgca gagcccaaat cttgtgac 28
<210> 6
   <211> 34
   <212> DNA
   <213> Artificial Sequence
<220>
   <223> primer, hIgG-R-XbaI
<400> 6
   gctctagagc tcatttaccc ggagacaggg agag 34
<210> 7
   <211> 1503
   <212> DNA
   <213> Homo sapiens
<220>
   <221> sig_peptide
   <222> (1)..(66)
<220>
   <221> CDS
   <222> (1)..(1500)
   <223> LAG3/Fc
<400> 7
   atg tgg gag gct cag ttc ctg ggc ttg ctg ttt ctg cag ccg ctt tgg      48
   Met Trp Glu Ala Gln Phe Leu Gly Leu Leu Phe Leu Gln Pro Leu Trp
    1               5                  10                  15
   gtg gct cca gtg aag cct ctc cag cca ggg gct gag gtc ccg gtg gtg      96
   Val Ala Pro Val Lys Pro Leu Gln Pro Gly Ala Glu Val Pro Val Val
               20                  25                  30
   tgg gcc cag gag ggg gct cct gcc cag ctc ccc tgc agc ccc aca atc         144
   Trp Ala Gln Glu Gly Ala Pro Ala Gln Leu Pro Cys Ser Pro Thr Ile
           35                  40                  45
   ccc ctc cag gat ctc agc ctt ctg cga aga gca ggg gtc act tgg cag         192
   Pro Leu Gln Asp Leu Ser Leu Leu Arg Arg Ala Gly Val Thr Trp Gln
       50                  55                  60
   cat cag cca gac agt ggc ccg ccc gct gcc gcc ccc ggc cat ccc ctg         240
   His Gln Pro Asp Ser Gly Pro Pro Ala Ala Ala Pro Gly His Pro Leu
   65                  70                  75                  80
   gcc ccc ggc cct cac ccg gcg gcg ccc tcc tcc tgg ggg ccc agg ccc         288
   Ala Pro Gly Pro His Pro Ala Ala Pro Ser Ser Trp Gly Pro Arg Pro
                   85                  90                  95
   cgc cgc tac acg gtg ctg agc gtg ggt ccc gga ggc ctg cgc agc ggg         336
   Arg Arg Tyr Thr Val Leu Ser Val Gly Pro Gly Gly Leu Arg Ser Gly
               100                 105                 110
   agg ctg ccc ctg cag ccc cgc gtc cag ctg gat gag cgc ggc cgg cag         384
   Arg Leu Pro Leu Gln Pro Arg Val Gln Leu Asp Glu Arg Gly Arg Gln
           115                 120                 125
   cgc ggg gac ttc tcg cta tgg ctg cgc cca gcc cgg cgc gcg gac gcc         432
   Arg Gly Asp Phe Ser Leu Trp Leu Arg Pro Ala Arg Arg Ala Asp Ala
       130                 135                 140
   ggc gag tac cgc gcc gcg gtg cac ctc agg gac cgc gcc ctc tcc tgc         480
   Gly Glu Tyr Arg Ala Ala Val His Leu Arg Asp Arg Ala Leu Ser Cys
   145                 150                 155                 160
   cgc ctc cgt ctg cgc ctg ggc cag gcc tcg atg act gcc agc ccc cca         528
   Arg Leu Arg Leu Arg Leu Gly Gln Ala Ser Met Thr Ala Ser Pro Pro
                   165                 170                 175
   gga tct ctc aga gcc tcc gac tgg gtc att ttg aac tgc tcc ttc agc         576
   Gly Ser Leu Arg Ala Ser Asp Trp Val Ile Leu Asn Cys Ser Phe Ser
               180                 185                 190
   cgc cct gac cgc cca gcc tct gtg cat tgg ttc cgg aac cgg ggc cag          624
   Arg Pro Asp Arg Pro Ala Ser Val His Trp Phe Arg Asn Arg Gly Gln
           195                 200                 205
   ggc cga gtc cct gtc cgg gag tcc ccc cat cac cac tta gcg gaa agc          672
   Gly Arg Val Pro Val Arg Glu Ser Pro His His His Leu Ala Glu Ser
       210                 215                 220
   ttc ctc ttc ctg ccc caa gtc agc ccc atg gac tct ggg ccc tgg ggc          720
   Phe Leu Phe Leu Pro Gln Val Ser Pro Met Asp Ser Gly Pro Trp Gly
   225                 230                 235                 240
   tgc atc ctc acc tac aga gat ggc ttc aac gtc tcc atc atg tat aac          768
   Cys Ile Leu Thr Tyr Arg Asp Gly Phe Asn Val Ser Ile Met Tyr Asn
                   245                 250                 255
   ctc act gtt ctg ggt ctg gag ccc cca act agt gca gag ccc aaa tct          816
   Leu Thr Val Leu Gly Leu Glu Pro Pro Thr Ser Ala Glu Pro Lys Ser
               260                 265                 270
   tgt gac aaa act cac aca tgc cca ccg tgc cca gca cct gaa ctc ctg          864
   Cys Asp Lys Thr His Thr Cys Pro Pro Cys Pro Ala Pro Glu Leu Leu
           275                 280                 285
   ggg gga ccg tca gtc ttc ctc ttc ccc cca aaa ccc aag gac acc ctc          912
   Gly Gly Pro Ser Val Phe Leu Phe Pro Pro Lys Pro Lys Asp Thr Leu
       290                 295                 300
   atg atc tcc cgg acc cct gag gtc aca tgc gtg gtg gtg gac gtg agc          960
   Met Ile Ser Arg Thr Pro Glu Val Thr Cys Val Val Val Asp Val Ser
   305                 310                 315                 320
   cac gaa gac cct gag gtc aag ttc aac tgg tac gtg gac ggc gtg gag         1008
   His Glu Asp Pro Glu Val Lys Phe Asn Trp Tyr Val Asp Gly Val Glu
                   325                 330                 335
   gtg cat aat gcc aag aca aag ccg cgg gag gag cag tac aac agc acg         1056
   Val His Asn Ala Lys Thr Lys Pro Arg Glu Glu Gln Tyr Asn Ser Thr
               340                 345                 350
   tac cgt gtg gtc agc gtc ctc acc gtc ctg cac cag gac tgg ctg aat        1104
   Tyr Arg Val Val Ser Val Leu Thr Val Leu His Gln Asp Trp Leu Asn
           355                 360                 365
   ggc aag gag tac aag tgc aag gtc tcc aac aaa gcc ctc cca gcc ccc        1152
   Gly Lys Glu Tyr Lys Cys Lys Val Ser Asn Lys Ala Leu Pro Ala Pro
       370                 375                 380
   atc gag aaa acc atc tcc aaa gcc aaa ggg cag ccc cga gaa cca cag        1200
   Ile Glu Lys Thr Ile Ser Lys Ala Lys Gly Gln Pro Arg Glu Pro Gln
   385                 390                 395                 400
   gtg tac acc ctg ccc cca tcc cgg gag gag atg acc aag aac cag gtc        1248
   Val Tyr Thr Leu Pro Pro Ser Arg Glu Glu Met Thr Lys Asn Gln Val
                   405                 410                 415
   agc ctg acc tgc ctg gtc aaa ggc ttc tat ccc agc gac atc gcc gtg        1296
   Ser Leu Thr Cys Leu Val Lys Gly Phe Tyr Pro Ser Asp Ile Ala Val
               420                 425                 430
   gag tgg gag agc aat ggg cag ccg gag aac aac tac aag acc acg cct        1344
   Glu Trp Glu Ser Asn Gly Gln Pro Glu Asn Asn Tyr Lys Thr Thr Pro
           435                 440                 445
   ccc gtg ctg gac tcc gac ggc tcc ttc ttc ctc tat agc aag ctc acc        1392
   Pro Val Leu Asp Ser Asp Gly Ser Phe Phe Leu Tyr Ser Lys Leu Thr
       450                 455                 460
   gtg gac aag agc agg tgg cag cag ggg aac gtc ttc tca tgc tcc gtg        1440
   Val Asp Lys Ser Arg Trp Gln Gln Gly Asn Val Phe Ser Cys Ser Val
   465                 470                 475                 480
   atg cat gag gct ctg cac aac cac tac acg cag aag agc ctc tcc ctg        1488
   Met His Glu Ala Leu His Asn His Tyr Thr Gln Lys Ser Leu Ser Leu
                   485                 490                 495
   tcc ccg ggt aaa      tga                                               1503
   Ser Pro Gly Lys
               500
<210> 8
   <211> 500
   <212> PRT
   <213> Homo sapiens
<400> 8
   Met Trp Glu Ala Gln Phe Leu Gly Leu Leu Phe Leu Gln Pro Leu Trp
    1               5                  10                  15
   Val Ala Pro Val Lys Pro Leu Gln Pro Gly Ala Glu Val Pro Val Val
               20                  25                  30
   Trp Ala Gln Glu Gly Ala Pro Ala Gln Leu Pro Cys Ser Pro Thr Ile
           35                  40                  45
   Pro Leu Gln Asp Leu Ser Leu Leu Arg Arg Ala Gly Val Thr Trp Gln
       50                  55                  60
   His Gln Pro Asp Ser Gly Pro Pro Ala Ala Ala Pro Gly His Pro Leu
   65                  70                  75                  80
   Ala Pro Gly Pro His Pro Ala Ala Pro Ser Ser Trp Gly Pro Arg Pro
                   85                  90                  95
   Arg Arg Tyr Thr Val Leu Ser Val Gly Pro Gly Gly Leu Arg Ser Gly
               100                 105                 110
   Arg Leu Pro Leu Gln Pro Arg Val Gln Leu Asp Glu Arg Gly Arg Gln
           115                 120                 125
   Arg Gly Asp Phe Ser Leu Trp Leu Arg Pro Ala Arg Arg Ala Asp Ala
       130                 135                 140
   Gly Glu Tyr Arg Ala Ala Val His Leu Arg Asp Arg Ala Leu Ser Cys
   145                 150                 155                 160
   Arg Leu Arg Leu Arg Leu Gly Gln Ala Ser Met Thr Ala Ser Pro Pro
                   165                 170                 175
   Gly Ser Leu Arg Ala Ser Asp Trp Val Ile Leu Asn Cys Ser Phe Ser
               180                 185                 190
   Arg Pro Asp Arg Pro Ala Ser Val His Trp Phe Arg Asn Arg Gly Gln
           195                 200                 205
   Gly Arg Val Pro Val Arg Glu Ser Pro His His His Leu Ala Glu Ser
       210                 215                 220
   Phe Leu Phe Leu Pro Gln Val Ser Pro Met Asp Ser Gly Pro Trp Gly
   225                 230                 235                 240
   Cys Ile Leu Thr Tyr Arg Asp Gly Phe Asn Val Ser Ile Met Tyr Asn
                   245                 250                 255
   Leu Thr Val Leu Gly Leu Glu Pro Pro Thr Ser Ala Glu Pro Lys Ser
               260                 265                 270
   Cys Asp Lys Thr His Thr Cys Pro Pro Cys Pro Ala Pro Glu Leu Leu
           275                 280                 285
   Gly Gly Pro Ser Val Phe Leu Phe Pro Pro Lys Pro Lys Asp Thr Leu
       290                 295                 300
   Met Ile Ser Arg Thr Pro Glu Val Thr Cys Val Val Val Asp Val Ser
   305                 310                 315                 320
   His Glu Asp Pro Glu Val Lys Phe Asn Trp Tyr Val Asp Gly Val Glu
                   325                 330                 335
   Val His Asn Ala Lys Thr Lys Pro Arg Glu Glu Gln Tyr Asn Ser Thr
               340                 345                 350
   Tyr Arg Val Val Ser Val Leu Thr Val Leu His Gln Asp Trp Leu Asn
           355                 360                 365
   Gly Lys Glu Tyr Lys Cys Lys Val Ser Asn Lys Ala Leu Pro Ala Pro
       370                 375                 380
   Ile Glu Lys Thr Ile Ser Lys Ala Lys Gly Gln Pro Arg Glu Pro Gln
   385 390 395 400
   Val Tyr Thr Leu Pro Pro Ser Arg Glu Glu Met Thr Lys Asn Gln Val
   405 410 415
   Ser Leu Thr Cys Leu Val Lys Gly Phe Tyr Pro Ser Asp Ile Ala Val
   420 425 430
   Glu Trp Glu Ser Asn Gly Gln Pro Glu Asn Asn Tyr Lys Thr Thr Pro
   435 440 445
   Pro Val Leu Asp Ser Asp Gly Ser Phe Phe Leu Tyr Ser Lys Leu Thr
   450 455 460
   Val Asp Lys Ser Arg Trp Gln Gln Gly Asn Val Phe Ser Cys Ser Val
   465 470 475 480
   Met His Glu Ala Leu His Asn His Tyr Thr Gln Lys Ser Leu Ser Leu
   485 490 495
   Ser Pro Gly Lys
   500
<210> 9
   <211> 2211
   <212> DNA
   <213> Homo sapiens
<220>
   <221> sig_peptide
   <222> (1)..(66)
<220>
   <221> CDS
   <222> (1)..(2208)
   <223> LAG3-LAG3/Fc
<400> 9
   atg tgg gag gct cag ttc ctg ggc ttg ctg ttt ctg cag ccg ctt tgg      48
   gtg gct cca gtg aag cct ctc cag cca ggg gct gag gtc ccg gtg gtg      96
   tgg gcc cag gag ggg gct cct gcc cag ctc ccc tgc agc ccc aca atc     144
   ccc ctc cag gat ctc agc ctt ctg cga aga gca ggg gtc act tgg cag     192
   cat cag cca gac agt ggc ccg ccc gct gcc gcc ccc ggc cat ccc ctg     240
   gcc ccc ggc cct cac ccg gcg gcg ccc tcc tcc tgg ggg ccc agg ccc     288
   cgc cgc tac acg gtg ctg agc gtg ggt ccc gga ggc ctg cgc agc ggg     336
   agg ctg ccc ctg cag ccc cgc gtc cag ctg gat gag cgc ggc cgg cag     384
   cgc ggg gac ttc tcg cta tgg ctg cgc cca gcc cgg cgc gcg gac gcc     432
   ggc gag tac cgc gcc gcg gtg cac ctc agg gac cgc gcc ctc tcc tgc     480
   cgc ctc cgt ctg cgc ctg ggc cag gcc tcg atg act gcc agc ccc cca     528
   gga tct ctc aga gcc tcc gac tgg gtc att ttg aac tgc tcc ttc agc     576
   cgc cct gac cgc cca gcc tct gtg cat tgg ttc cgg aac cgg ggc cag     624
   ggc cga gtc cct gtc cgg gag tcc ccc cat cac cac tta gcg gaa agc     672
   ttc ctc ttc ctg ccc caa gtc agc ccc atg gac tct ggg ccc tgg ggc     720
   tgc atc ctc acc tac aga gat ggc ttc aac gtc tcc atc atg tat aac     768
   ctc act ctc cag cca ggg gct gag gtc ccg gtg gtg tgg gcc cag gag     816
   ggg gct cct gcc cag ctc ccc tgc agc ccc aca atc ccc ctc cag gat     864
   ctc agc ctt ctg cga aga gca ggg gtc act tgg cag cat cag cca gac     912
   agt ggc ccg ccc gct gcc gcc ccc ggc cat ccc ctg gcc ccc ggc cct     960
   cac ccg gcg gcg ccc tcc tcc tgg ggg ccc agg ccc cgc cgc tac acg        1008
   gtg ctg agc gtg ggt ccc gga ggc ctg cgc agc ggg agg ctg ccc ctg        1056
   cag ccc cgc gtc cag ctg gat gag cgc ggc cgg cag cgc ggg gac ttc        1104
   tcg cta tgg ctg cgc cca gcc cgg cgc gcg gac gcc ggc gag tac cgc        1152
   gcc gcg gtg cac ctc agg gac cgc gcc ctc tcc tgc cgc ctc cgt ctg        1200
   cgc ctg ggc cag gcc tcg atg act gcc agc ccc cca gga tct ctc aga        1248
   gcc tcc gac tgg gtc att ttg aac tgc tcc ttc agc cgc cct gac cgc        1296
   cca gcc tct gtg cat tgg ttc cgg aac cgg ggc cag ggc cga gtc cct        1344
   gtc cgg gag tcc ccc cat cac cac tta gcg gaa agc ttc ctc ttc ctg        1392
   ccc caa gtc agc ccc atg gac tct ggg ccc tgg ggc tgc atc ctc acc        1440
   tac aga gat ggc ttc aac gtc tcc atc atg tat aac ctc act gtt ctg         1488
   ggt ctg gag ccc cca act agt gca gag ccc aaa tct tgt gac aaa act         1536
   cac aca tgc cca ccg tgc cca gca cct gaa ctc ctg ggg gga ccg tca         1584
   gtc ttc ctc ttc ccc cca aaa ccc aag gac acc ctc atg atc tcc cgg         1632
   acc cct gag gtc aca tgc gtg gtg gtg gac gtg agc cac gaa gac cct         1680
   gag gtc aag ttc aac tgg tac gtg gac ggc gtg gag gtg cat aat gcc         1728
   aag aca aag ccg cgg gag gag cag tac aac agc acg tac cgt gtg gtc         1776
   agc gtc ctc acc gtc ctg cac cag gac tgg ctg aat ggc aag gag tac         1824
   aag tgc aag gtc tcc aac aaa gcc ctc cca gcc ccc atc gag aaa acc         1872
   atc tcc aaa gcc aaa ggg cag ccc cga gaa cca cag gtg tac acc ctg         1920
   ccc cca tcc cgg gag gag atg acc aag aac cag gtc agc ctg acc tgc        1968
   ctg gtc aaa ggc ttc tat ccc agc gac atc gcc gtg gag tgg gag agc        2016
   aat ggg cag ccg gag aac aac tac aag acc acg cct ccc gtg ctg gac        2064
   tcc gac ggc tcc ttc ttc ctc tat agc aag ctc acc gtg gac aag agc        2112
   agg tgg cag cag ggg aac gtc ttc tca tgc tcc gtg atg cat gag gct        2160
   ctg cac aac cac tac acg cag aag agc ctc tcc ctg tcc ccg ggt aaa        2208
   tg a                                                           2211
<210> 10
   <211> 736
   <212> PRT
   <213> Homo sapiens
<400> 10
<210> 11
   <211> 1473
   <212> DNA
   <213> Homo sapiens
<220>
   <221> CDS
   <222> (1)..(1470)
   <223> TNFR2/Fc
<400> 11
   atg gcg ccc gtc gcc gtc tgg gcc gcg ctg gcc gtc gga ctg gag ctc 48
   Met Ala Pro Val Ala Val Trp Ala Ala Leu Ala Val Gly Leu Glu Leu
   1 5 10 15
   tgg gct gcg gcg cac gcc ttg ccc gcc cag gtg gca ttt aca ccc tac 96
   Trp Ala Ala Ala His Ala Leu Pro Ala Gln Val Ala Phe Thr Pro Tyr
   20 25 30
   gcc ccg gag ccc ggg agc aca tgc cgg ctc aga gaa tac tat gac cag 144
   Ala Pro Glu Pro Gly Ser Thr Cys Arg Leu Arg Glu Tyr Tyr Asp Gln
   35 40 45
   aca gct cag atg tgc tgc agc aaa tgc tcg ccg ggc caa cat gca aaa 192
   Thr Ala Gln Met Cys Cys Ser Lys Cys Ser Pro Gly Gln His Ala Lys
   50 55 60
   gtc ttc tgt acc aag acc tcg gac acc gtg tgt gac tcc tgt gag gac 240
   Val Phe Cys Thr Lys Thr Ser Asp Thr Val Cys Asp Ser Cys Glu Asp
   65 70 75 80
   agc aca tac acc cag ctc tgg aac tgg gtt ccc gag tgc ttg agc tgt 288
   Ser Thr Tyr Thr Gln Leu Trp Asn Trp Val Pro Glu Cys Leu Ser Cys
   85 90 95
   ggc tcc cgc tgt agc tct gac cag gtg gaa act caa gcc tgc act cgg 336
   Gly Ser Arg Cys Ser Ser Asp Gln Val Glu Thr Gln Ala Cys Thr Arg
   100 105 110
   gaa cag aac cgc atc tgc acc tgc agg ccc ggc tgg tac tgc gcg ctg 384
   Glu Gln Asn Arg Ile Cys Thr Cys Arg Pro Gly Trp Tyr Cys Ala Leu
   115 120 125
   agc aag cag gag ggg tgc cgg ctg tgc gcg ccg ctg cgc aag tgc cgc 432
   Ser Lys Gln Glu Gly Cys Arg Leu Cys Ala Pro Leu Arg Lys Cys Arg
   130 135 140
   ccg ggc ttc ggc gtg gcc aga cca gga act gaa aca tca gac gtg gtg 480
   Pro Gly Phe Gly Val Ala Arg Pro Gly Thr Glu Thr Ser Asp Val Val
   145 150 155 160
   tgc aag ccc tgt gcc ccg ggg acg ttc tcc aac acg act tca tcc acg 528
   Cys Lys Pro Cys Ala Pro Gly Thr Phe Ser Asn Thr Thr Ser Ser Thr
   165 170 175
   gat att tgc agg ccc cac cag atc tgt aac gtg gtg gcc atc cct ggg 576
   Asp Ile Cys Arg Pro His Gln Ile Cys Asn Val Val Ala Ile Pro Gly
   180 185 190
   aat gca agc atg gat gca gtc tgc acg tcc acg tcc ccc acc cgg agt 624
   Asn Ala Ser Met Asp Ala Val Cys Thr Ser Thr Ser Pro Thr Arg Ser
   195 200 205
   atg gcc cca ggg gca gta cac tta ccc cag cca gtg tcc aca cga tcc 672
   Met Ala Pro Gly Ala Val His Leu Pro Gln Pro Val Ser Thr Arg Ser
       210                 215                 220
   caa cac acg cag cca act cca gaa ccc agc act gct cca agc acc tcc         720
   Gln His Thr Gln Pro Thr Pro Glu Pro Ser Thr Ala Pro Ser Thr Ser
   225                 230                 235                 240
   ttc ctg ctc cca atg ggc ccc agc ccc cca gct gaa ggg agc act ggc         768
   Phe Leu Leu Pro Met Gly Pro Ser Pro Pro Ala Glu Gly Ser Thr Gly
                   245                 250                 255
   gac gca gag ccc aaa tct tgt gac aaa act cac aca tgc cca ccg tgc         816
   Asp Ala Glu Pro Lys Ser Cys Asp Lys Thr His Thr Cys Pro Pro Cys
               260                 265                 270
   cca gca cct gaa ctc ctg ggg gga ccg tca gtc ttc ctc ttc ccc cca         864
   Pro Ala Pro Glu Leu Leu Gly Gly Pro Ser Val Phe Leu Phe Pro Pro
           275                 280                 285
   aaa ccc aag gac acc ctc atg atc tcc cgg acc cct gag gtc aca tgc         912
   Lys Pro Lys Asp Thr Leu Met Ile Ser Arg Thr Pro Glu Val Thr Cys
       290                 295                 300
   gtg gtg gtg gac gtg agc cac gaa gac cct gag gtc aag ttc aac tgg         960
   Val Val Val Asp Val Ser His Glu Asp Pro Glu Val Lys Phe Asn Trp
   305                 310                 315                 320
   tac gtg gac ggc gtg gag gtg cat aat gcc aag aca aag ccg cgg gag        1008
   Tyr Val Asp Gly Val Glu Val His Asn Ala Lys Thr Lys Pro Arg Glu
                   325                 330                 335
   gag cag tac aac agc acg tac cgg gtg gtc agc gtc ctc acc gtc ctg        1056
   Glu Gln Tyr Asn Ser Thr Tyr Arg Val Val Ser Val Leu Thr Val Leu
               340                 345                 350
   cac cag gac tgg ctg aat ggc aag gag tac aag tgc aag gtc tcc aac        1104
   His Gln Asp Trp Leu Asn Gly Lys Glu Tyr Lys Cys Lys Val Ser Asn
           355                 360                 365
   aaa gcc ctc cca gcc ccc atc gag aaa acc atc tcc aaa gcc aaa ggg        1152
   Lys Ala Leu Pro Ala Pro Ile Glu Lys Thr Ile Ser Lys Ala Lys Gly
           370                 375                 380
   cag ccc cga gaa cca cag gtg tac acc ctg ccc cca tcc cgg gat gag        1200
   Gln Pro Arg Glu Pro Gln Val Tyr Thr Leu Pro Pro Ser Arg Asp Glu
   385                 390                 395                 400
   ctg acc aag aac cag gtc agc ctg acc tgc ctg gtc aaa ggc ttc tat        1248
   Leu Thr Lys Asn Gln Val Ser Leu Thr Cys Leu Val Lys Gly Phe Tyr
                   405                 410                 415
   ccc agc gac atc gcc gtg gag tgg gag agc aat ggg cag ccg gag aac        1296
   Pro Ser Asp Ile Ala Val Glu Trp Glu Ser Asn Gly Gln Pro Glu Asn
               420                 425                 430
   aac tac aag acc acg cct ccc gtg ctg gac tcc gac ggc tcc tcc ttc        1344
   Asn Tyr Lys Thr Thr Pro Pro Val Leu Asp Ser Asp Gly Ser Ser Phe
           435                 440                 445
   ctc tac agc aag ctc acc gtg gac aag agc agg tgg cag cag ggg aac        1392
   Leu Tyr Ser Lys Leu Thr Val Asp Lys Ser Arg Trp Gln Gln Gly Asn
       450                 455                 460
   gtc ttc tca tgc tcc gtg atg cat gag gct ctg cac aac cac tac acg        1440
   Val Phe Ser Cys Ser Val Met His Glu Ala Leu His Asn His Tyr Thr
   465                 470                 475                 480
   cag aag agc ctc tcc ctg tct ccg ggt aaa     tga                        1473
   Gln Lys Ser Leu Ser Leu Ser Pro Gly Lys
                   485                 490
<210> 12
   <211> 490
   <212> PRT
   <213> Homo sapiens
<400> 12
   Met Ala Pro Val Ala Val Trp Ala Ala Leu Ala Val Gly Leu Glu Leu
    1               5                  10                  15
   Trp Ala Ala Ala His Ala Leu Pro Ala Gln Val Ala Phe Thr Pro Tyr
   20 25 30
   Ala Pro Glu Pro Gly Ser Thr Cys Arg Leu Arg Glu Tyr Tyr Asp Gln
   35 40 45
   Thr Ala Gln Met Cys Cys Ser Lys Cys Ser Pro Gly Gln His Ala Lys
   50 55 60
   Val Phe Cys Thr Lys Thr Ser Asp Thr Val Cys Asp Ser Cys Glu Asp
   65 70 75 80
   Ser Thr Tyr Thr Gln Leu Trp Asn Trp Val Pro Glu Cys Leu Ser Cys
   85 90 95
   Gly Ser Arg Cys Ser Ser Asp Gln Val Glu Thr Gln Ala Cys Thr Arg
   100 105 110
   Glu Gln Asn Arg Ile Cys Thr Cys Arg Pro Gly Trp Tyr Cys Ala Leu
   115 120 125
   Ser Lys Gln Glu Gly Cys Arg Leu Cys Ala Pro Leu Arg Lys Cys Arg
   130 135 140
   Pro Gly Phe Gly Val Ala Arg Pro Gly Thr Glu Thr Ser Asp Val Val
   145 150 155 160
   Cys Lys Pro Cys Ala Pro Gly Thr Phe Ser Asn Thr Thr Ser Ser Thr
   165 170 175
   Asp Ile Cys Arg Pro His Gln Ile Cys Asn Val Val Ala Ile Pro Gly
   180 185 190
   Asn Ala Ser Met Asp Ala Val Cys Thr Ser Thr Ser Pro Thr Arg Ser
   195 200 205
   Met Ala Pro Gly Ala Val His Leu Pro Gln Pro Val Ser Thr Arg Ser
   210 215 220
   Gln His Thr Gln Pro Thr Pro Glu Pro Ser Thr Ala Pro Ser Thr Ser
   225                 230                 235                 240
   Phe Leu Leu Pro Met Gly Pro Ser Pro Pro Ala Glu Gly Ser Thr Gly
                   245                 250                 255
   Asp Ala Glu Pro Lys Ser Cys Asp Lys Thr His Thr Cys Pro Pro Cys
               260                 265                 270
   Pro Ala Pro Glu Leu Leu Gly Gly Pro Ser Val Phe Leu Phe Pro Pro
           275                 280                 285
   Lys Pro Lys Asp Thr Leu Met Ile Ser Arg Thr Pro Glu Val Thr Cys
       290                 295                 300
   Val Val Val Asp Val Ser His Glu Asp Pro Glu Val Lys Phe Asn Trp
   305                 310                 315                 320
   Tyr Val Asp Gly Val Glu Val His Asn Ala Lys Thr Lys Pro Arg Glu
                   325                 330                 335
   Glu Gln Tyr Asn Ser Thr Tyr Arg Val Val Ser Val Leu Thr Val Leu
               340                 345                 350
   His Gln Asp Trp Leu Asn Gly Lys Glu Tyr Lys Cys Lys Val Ser Asn
           355                 360                 365
   Lys Ala Leu Pro Ala Pro Ile Glu Lys Thr Ile Ser Lys Ala Lys Gly
       370                 375                 380
   Gln Pro Arg Glu Pro Gln Val Tyr Thr Leu Pro Pro Ser Arg Asp Glu
   385                 390                 395                 400
   Leu Thr Lys Asn Gln Val Ser Leu Thr Cys Leu Val Lys Gly Phe Tyr
                   405                 410                 415
   Pro Ser Asp Ile Ala Val Glu Trp Glu Ser Asn Gly Gln Pro Glu Asn
               420                 425                 430
   Asn Tyr Lys Thr Thr Pro Pro Val Leu Asp Ser Asp Gly Ser Ser Phe
           435                 440                 445
   Leu Tyr Ser Lys Leu Thr Val Asp Lys Ser Arg Trp Gln Gln Gly Asn
       450                 455                 460
   Val Phe Ser Cys Ser Val Met His Glu Ala Leu His Asn His Tyr Thr
   465                 470                 475                 480
   Gln Lys Ser Leu Ser Leu Ser Pro Gly Lys
                   485                 490
<210> 13
   <211> 2163
   <212> DNA
   <213> Homo sapiens
<220>
   <221> CDS
   <222> (1)..(2160)
   <223> TNFR2-TNFR2/Fc
<400> 13
   atg gcg ccc gtc gcc gtc tgg gcc gcg ctg gcc gtc gga ctg gag ctc         48
   tgg gct gcg gcg cac gcc ttg ccc gcc cag gtg gca ttt aca ccc tac         96
   gcc ccg gag ccc ggg agc aca tgc cgg ctc aga gaa tac tat gac cag        144
   aca gct cag atg tgc tgc agc aaa tgc tcg ccg ggc caa cat gca aaa        192
   gtc ttc tgt acc aag acc tcg gac acc gtg tgt gac tcc tgt gag gac        240
   agc aca tac acc cag ctc tgg aac tgg gtt ccc gag tgc ttg agc tgt        288
   ggc tcc cgc tgt agc tct gac cag gtg gaa act caa gcc tgc act cgg        336
   gaa cag aac cgc atc tgc acc tgc agg ccc ggc tgg tac tgc gcg ctg        384
   agc aag cag gag ggg tgc cgg ctg tgc gcg ccg ctg cgc aag tgc cgc        432
   ccg ggc ttc ggc gtg gcc aga cca gga act gaa aca tca gac gtg gtg        480
   tgc aag ccc tgt gcc ccg ggg acg ttc tcc aac acg act tca tcc acg        528
   gat att tgc agg ccc cac cag atc tgt aac gtg gtg gcc atc cct ggg        576
   aat gca agc atg gat gca gtc tgc acg tcc acg tcc ccc acc cgg agt        624
   atg gcc cca ggg gca gta cac tta ccc cag cca gtg tcc aca cga tcc        672
   caa cac acg cag cca act cca gaa ccc agc act gct cca agc acc tcc        720
   ttc ctg ctc cca atg ggc ccc agc ccc cca gct gaa ggg agc gga tcc         768
   aac gca act aca ccc tac gcc ccg gag ccc ggg agc aca tgc cgg ctc         816
   aga gaa tac tat gac cag aca gct cag atg tgc tgc agc aaa tgc tcg         864
   ccg ggc caa cat gca aaa gtc ttc tgt acc aag acc tcg gac acc gtg         912
   tgt gac tcc tgt gag gac agc aca tac acc cag ctc tgg aac tgg gtt         960
   ccc gag tgc ttg agc tgt ggc tcc cgc tgt agc tct gac cag gtg gaa        1008
   act caa gcc tgc act cgg gaa cag aac cgc atc tgc acc tgc agg ccc        1056
   ggc tgg tac tgc gcg ctg agc aag cag gag ggg tgc cgg ctg tgc gcg        1104
   ccg ctg cgc aag tgc cgc ccg ggc ttc ggc gtg gcc aga cca gga act        1152
   gaa aca tca gac gtg gtg tgc aag ccc tgt gcc ccg ggg acg ttc tcc        1200
   aac acg act tca tcc acg gat att tgc agg ccc cac cag atc tgt aac       1248
   gtg gtg gcc atc cct ggg aat gca agc atg gat gca gtc tgc acg tcc       1296
   acg tcc ccc acc cgg agt atg gcc cca ggg gca gta cac tta ccc cag       1344
   cca gtg tcc aca cga tcc caa cac acg cag cca act cca gaa ccc agc       1392
   act gct cca agc acc tcc ttc ctg ctc cca atg ggc ccc agc ccc cca       1440
   gct gaa ggg agc act ggc gac gca gag ccc aaa tct tgt gac aaa act       1488
   cac aca tgc cca ccg tgc cca gca cct gaa ctc ctg ggg gga ccg tca       1536
   gtc ttc ctc ttc ccc cca aaa ccc aag gac acc ctc atg atc tcc cgg       1584
   acc cct gag gtc aca tgc gtg gtg gtg gac gtg agc cac gaa gac cct       1632
   gag gtc aag ttc aac tgg tac gtg gac ggc gtg gag gtg cat aat gcc       1680
   aag aca aag ccg cgg gag gag cag tac aac agc acg tac cgg gtg gtc 1728
   agc gtc ctc acc gtc ctg cac cag gac tgg ctg aat ggc aag gag tac 1776
   aag tgc aag gtc tcc aac aaa gcc ctc cca gcc ccc atc gag aaa acc 1824
   atc tcc aaa gcc aaa ggg cag ccc cga gaa cca cag gtg tac acc ctg 1872
   ccc cca tcc cgg gat gag ctg acc aag aac cag gtc agc ctg acc tgc 1920
   ctg gtc aaa ggc ttc tat ccc agc gac atc gcc gtg gag tgg gag agc 1968
   aat ggg cag ccg gag aac aac tac aag acc acg cct ccc gtg ctg gac 2016
   tcc gac ggc tcc tcc ttc ctc tac agc aag ctc acc gtg gac aag agc 2064
   agg tgg cag cag ggg aac gtc ttc tca tgc tcc gtg atg cat gag gct 2112
   ctg cac aac cac tac acg cag aag agc ctc tcc ctg tct ccg ggt aaa 2160
   tga                                                                     2163
<210> 14
   <211> 720
   <212> PRT
   <213> Homo sapiens
<400> 14
<210> 15
   <211> 1314
   <212> DNA
   <213> Homo sapiens
<220>
   <221> CDS
   <222> (1)..(1311)
   <223> CD2/Fc
<400> 15
   atg agc ttt cca tgt aaa ttt gta gcc agc ttc ctt ctg att ttc aat        48
   Met Ser Phe Pro Cys Lys Phe Val Ala Ser Phe Leu Leu Ile Phe Asn
    1               5                  10                  15
   gtt tct tcc aaa ggt gca gtc tcc aaa gag att acg aat gcc ttg gaa        96
   Val Ser Ser Lys Gly Ala Val Ser Lys Glu Ile Thr Asn Ala Leu Glu
               20                  25                  30
   acc tgg ggt gcc ttg ggt cag gac atc aac ttg gac att cct agt ttt       144
   Thr Trp Gly Ala Leu Gly Gln Asp Ile Asn Leu Asp Ile Pro Ser Phe
           35                  40                  45
   caa atg agt gat gat att gac gat ata aaa tgg gaa aaa act tca gac       192
   Gln Met Ser Asp Asp Ile Asp Asp Ile Lys Trp Glu Lys Thr Ser Asp
       50                  55                  60
   aag aaa aag att gca caa ttc aga aaa gag aaa gag act ttc aag gaa       240
   Lys Lys Lys Ile Ala Gln Phe Arg Lys Glu Lys Glu Thr Phe Lys Glu
   65                  70                  75                  80
   aaa gat aca tat aag cta ttt aaa aat gga act ctg aaa att aag cat       288
   Lys Asp Thr Tyr Lys Leu Phe Lys Asn Gly Thr Leu Lys Ile Lys His
                   85                  90                  95
   ctg aag acc gat gat cag gat atc tac aag gta tca ata tat gat aca       336
   Leu Lys Thr Asp Asp Gln Asp Ile Tyr Lys Val Ser Ile Tyr Asp Thr
               100                 105                 110
   aaa gga aaa aat gtg ttg gaa aaa ata ttt gat ttg aag att caa gag       384
   Lys Gly Lys Asn Val Leu Glu Lys Ile Phe Asp Leu Lys Ile Gln Glu
           115                 120                 125
   agg gtc tca aaa cca aag atc tcc tgg act tgt atc aac aca acc ctg       432
   Arg Val Ser Lys Pro Lys Ile Ser Trp Thr Cys Ile Asn Thr Thr Leu
       130                 135                 140
   acc tgt gag gta atg aat gga act gac ccc gaa tta aac ctg tat caa       480
   Thr Cys Glu Val Met Asn Gly Thr Asp Pro Glu Leu Asn Leu Tyr Gln
   145                 150                 155                 160
   gat ggg aaa cat cta aaa ctt tct cag agg gtc atc aca cac aag tgg        528
   Asp Gly Lys His Leu Lys Leu Ser Gln Arg Val Ile Thr His Lys Trp
                   165                 170                 175
   acc acc agc ctg agt gca aaa ttc aag tgc aca gca ggg aac aaa gtc        576
   Thr Thr Ser Leu Ser Ala Lys Phe Lys Cys Thr Ala Gly Asn Lys Val
               180                 185                 190
   agc aag gaa tcc agt gtc gag cct gtc agc tgt cct gca gag ccc aaa        624
   Ser Lys Glu Ser Ser Val Glu Pro Val Ser Cys Pro Ala Glu Pro Lys
           195                 200                 205
   tct tgt gac aaa act cac aca tgc cca ccg tgc cca gca cct gaa ctc        672
   Ser Cys Asp Lys Thr His Thr Cys Pro Pro Cys Pro Ala Pro Glu Leu
       210                 215                 220
   ctg ggg gga ccg tca gtc ttc ctc ttc ccc cca aaa ccc aag gac acc        720
   Leu Gly Gly Pro Ser Val Phe Leu Phe Pro Pro Lys Pro Lys Asp Thr
   225                 230                 235                 240
   ctc atg atc tcc cgg acc cct gag gtc aca tgc gtg gtg gtg gac gtg        768
   Leu Met Ile Ser Arg Thr Pro Glu Val Thr Cys Val Val Val Asp Val
                   245                 250                 255
   agc cac gaa gac cct gag gtc aag ttc aac tgg tac gtg gac ggc gtg        816
   Ser His Glu Asp Pro Glu Val Lys Phe Asn Trp Tyr Val Asp Gly Val
               260                 265                 270
   gag gtg cat aat gcc aag aca aag ccg cgg gag gag cag tac aac agc        864
   Glu Val His Asn Ala Lys Thr Lys Pro Arg Glu Glu Gln Tyr Asn Ser
           275                 280                 285
   acg tac cgg gtg gtc agc gtc ctc acc gtc ctg cac cag gac tgg ctg        912
   Thr Tyr Arg Val Val Ser Val Leu Thr Val Leu His Gln Asp Trp Leu
       290                 295                 300
   aat ggc aag gag tac aag tgc aag gtc tcc aac aaa gcc ctc cca gcc        960
   Asn Gly Lys Glu Tyr Lys Cys Lys Val Ser Asn Lys Ala Leu Pro Ala
   305                 310                 315                 320
   ccc atc gag aaa acc atc tcc aaa gcc aaa ggg cag ccc cga gaa cca         1008
   Pro Ile Glu Lys Thr Ile Ser Lys Ala Lys Gly Gln Pro Arg Glu Pro
                   325                 330                 335
   cag gtg tac acc ctg ccc cca tcc cgg gat gag ctg acc aag aac cag         1056
   Gln Val Tyr Thr Leu Pro Pro Ser Arg Asp Glu Leu Thr Lys Asn Gln
               340                 345                 350
   gtc agc ctg acc tgc ctg gtc aaa ggc ttc tat ccc agc gac atc gcc         1104
   Val Ser Leu Thr Cys Leu Val Lys Gly Phe Tyr Pro Ser Asp Ile Ala
           355                 360                 365
   gtg gag tgg gag agc aat ggg cag ccg gag aac aac tac aag acc acg         1152
   Val Glu Trp Glu Ser Asn Gly Gln Pro Glu Asn Asn Tyr Lys Thr Thr
       370                 375                 380
   cct ccc gtg ctg gac tcc gac ggc tcc ttc ttc ctc tac agc aag ctc         1200
   Pro Pro Val Leu Asp Ser Asp Gly Ser Phe Phe Leu Tyr Ser Lys Leu
   385                 390                 395                 400
   acc gtg gac aag agc agg tgg cag cag ggg aac gtc ttc tca tgc tcc         1248
   Thr Val Asp Lys Ser Arg Trp Gln Gln Gly Asn Val Phe Ser Cys Ser
                   405                 410                 415
   gtg atg cat gag gct ctg cac aac cac tac acg cag aag agc ctc tcc         1296
   Val Met His Glu Ala Leu His Asn His Tyr Thr Gln Lys Ser Leu Ser
               420                 425                 430
   ctg tct ccg ggt aaa    tga                                              1314
   Leu Ser Pro Gly Lys
           435
<210> 16
   <211> 437
   <212> PRT
   <213> Homo sapiens
<400> 16
   Met Ser Phe Pro Cys Lys Phe Val Ala Ser Phe Leu Leu Ile Phe Asn
   1               5                   10                  15
   Val Ser Ser Lys Gly Ala Val Ser Lys Glu Ile Thr Asn Ala Leu Glu
               20                  25                  30
   Thr Trp Gly Ala Leu Gly Gln Asp Ile Asn Leu Asp Ile Pro Ser Phe
           35                  40                  45
   Gln Met Ser Asp Asp Ile Asp Asp Ile Lys Trp Glu Lys Thr Ser Asp
       50                  55                  60
   Lys Lys Lys Ile Ala Gln Phe Arg Lys Glu Lys Glu Thr Phe Lys Glu
   65                  70                  75                  80
   Lys Asp Thr Tyr Lys Leu Phe Lys Asn Gly Thr Leu Lys Ile Lys His
                   85                  90                  95
   Leu Lys Thr Asp Asp Gln Asp Ile Tyr Lys Val Ser Ile Tyr Asp Thr
               100                 105                 110
   Lys Gly Lys Asn Val Leu Glu Lys Ile Phe Asp Leu Lys Ile Gln Glu
           115                 120                 125
   Arg Val Ser Lys Pro Lys Ile Ser Trp Thr Cys Ile Asn Thr Thr Leu
       130                 135                 140
   Thr Cys Glu Val Met Asn Gly Thr Asp Pro Glu Leu Asn Leu Tyr Gln
   145                 150                 155                 160
   Asp Gly Lys His Leu Lys Leu Ser Gln Arg Val Ile Thr His Lys Trp
                   165                 170                 175
   Thr Thr Ser Leu Ser Ala Lys Phe Lys Cys Thr Ala Gly Asn Lys Val
               180                 185                 190
   Ser Lys Glu Ser Ser Val Glu Pro Val Ser Cys Pro Ala Glu Pro Lys
           195                 200                 205
   Ser Cys Asp Lys Thr His Thr Cys Pro Pro Cys Pro Ala Pro Glu Leu
       210                 215                 220
   Leu Gly Gly Pro Ser Val Phe Leu Phe Pro Pro Lys Pro Lys Asp Thr
   225 230 235 240
   Leu Met Ile Ser Arg Thr Pro Glu Val Thr Cys Val Val Val Asp Val
   245 250 255
   Ser His Glu Asp Pro Glu Val Lys Phe Asn Trp Tyr Val Asp Gly Val
   260 265 270
   Glu Val His Asn Ala Lys Thr Lys Pro Arg Glu Glu Gln Tyr Asn Ser
   275 280 285
   Thr Tyr Arg Val Val Ser Val Leu Thr Val Leu His Gln Asp Trp Leu
   290 295 300
   Asn Gly Lys Glu Tyr Lys Cys Lys Val Ser Asn Lys Ala Leu Pro Ala
   305 310 315 320
   Pro Ile Glu Lys Thr Ile Ser Lys Ala Lys Gly Gln Pro Arg Glu Pro
   325 330 335
   Gln Val Tyr Thr Leu Pro Pro Ser Arg Asp Glu Leu Thr Lys Asn Gln
   340 345 350
   Val Ser Leu Thr Cys Leu Val Lys Gly Phe Tyr Pro Ser Asp Ile Ala
   355 360 365
   Val Glu Trp Glu Ser Asn Gly Gln Pro Glu Asn Asn Tyr Lys Thr Thr
   370 375 380
   Pro Pro Val Leu Asp Ser Asp Gly Ser Phe Phe Leu Tyr Ser Lys Leu
   385 390 395 400
   Thr Val Asp Lys Ser Arg Trp Gln Gln Gly Asn Val Phe Ser Cys Ser
   405 410 415
   Val Met His Glu Ala Leu His Asn His Tyr Thr Gln Lys Ser Leu Ser
   420 425 430
   Leu Ser Pro Gly Lys
   435
<210> 17
   <211> 1854
   <212> DNA
   <213> Homo sapiens
<220>
   <221> CDS
   <222> (1)..(1851)
   <223> CD2-CD2/Fc
<400> 17
   atg agc ttt cca tgt aaa ttt gta gcc agc ttc ctt ctg att ttc aat      48
   gtt tct tcc aaa ggt gca gtc tcc aaa gag att acg aat gcc ttg gaa      96
   acc tgg ggt gcc ttg ggt cag gac atc aac ttg gac att cct agt ttt     144
   caa atg agt gat gat att gac gat ata aaa tgg gaa aaa act tca gac     192
   aag aaa aag att gca caa ttc aga aaa gag aaa gag act ttc aag gaa     240
   aaa gat aca tat aag cta ttt aaa aat gga act ctg aaa att aag cat     288
   ctg aag acc gat gat cag gat atc tac aag gta tca ata tat gat aca         336
   aaa gga aaa aat gtg ttg gaa aaa ata ttt gat ttg aag att caa gag         384
   agg gtc tca aaa cca aag atc tcc tgg act tgt atc aac aca acc ctg         432
   acc tgt gag gta atg aat gga act gac ccc gaa tta aac ctg tat caa         480
   gat ggg aaa cat cta aaa ctt tct cag agg gtc atc aca cac aag tgg         528
   acc acc agc ctg agt gca aaa ttc aag tgc aca gca ggg aac aaa gtc         576
   agc aag gaa tcc agt gtc gag cct gtc agc tgt cct aaa gag att acg         624
   aat gcc ttg gaa acc tgg ggt gcc ttg ggt cag gac atc aac ttg gac         672
   att cct agt ttt caa atg agt gat gat att gac gat ata aaa tgg gaa         720
   aaa act tca gac aag aaa aag att gca caa ttc aga aaa gag aaa gag         768
   act ttc aag gaa aaa gat aca tat aag cta ttt aaa aat gga act ctg 816
   aaa att aag cat ctg aag acc gat gat cag gat atc tac aag gta tca 864
   ata tat gat aca aaa gga aaa aat gtg ttg gaa aaa ata ttt gat ttg 912
   aag att caa gag agg gtc tca aaa cca aag atc tcc tgg act tgt atc 960
   aac aca acc ctg acc tgt gag gta atg aat gga act gac ccc gaa tta 1008
   aac ctg tat caa gat ggg aaa cat cta aaa ctt tct cag agg gtc atc 1056
   aca cac aag tgg acc acc agc ctg agt gca aaa ttc aag tgc aca gca 1104
   ggg aac aaa gtc agc aag gaa tcc agt gtc gag cct gtc agc tgt cct 1152
   gca gag ccc aaa tct tgt gac aaa act cac aca tgc cca ccg tgc cca 1200
   gca cct gaa ctc ctg ggg gga ccg tca gtc ttc ctc ttc ccc cca aaa 1248
   ccc aag gac acc ctc atg atc tcc cgg acc cct gag gtc aca tgc gtg        1296
   gtg gtg gac gtg agc cac gaa gac cct gag gtc aag ttc aac tgg tac        1344
   gtg gac ggc gtg gag gtg cat aat gcc aag aca aag ccg cgg gag gag        1392
   cag tac aac agc acg tac cgg gtg gtc agc gtc ctc acc gtc tgt cac        1440
   cag gac tgg ctg aat ggc aag gag tac aag tgc aag gtc tcc aac aaa        1488
   gcc ctc cca gcc ccc atc gag aaa acc atc tcc aaa gcc aaa ggg cag        1536
   ccc cga gaa cca cag gtg tac acc ctg ccc cca tcc cgg gat gag ctg        1584
   acc aag aac cag gtc agc ctg acc tgc ctg gtc aaa ggc ttc tat ccc        1632
   agc gac atc gcc gtg gag tgg gag agc aat ggg cag ccg gag aac aac        1680
   tac aag acc acg cct ccc gtg ctg gac tcc gac ggc tcc ttc ttc ctc        1728
   tac agc aag ctc acc gtg gac aag agc agg tgg cag cag ggg aac gtc        1776
   ttc tca tgc tcc gtg atg cat gag gct ctg cac aac cac tac acg cag        1824
   aag agc ctc tcc ctg tct ccg ggt aaa        tga                         1854
<210> 18
   <211> 617
   <212> PRT
   <213> Homo sapiens
<400> 18
<210> 19
   <211> 1134
   <212> DNA
   <213> Homo sapiens
<220>
   <221> CDS
   <222> (1)..(1131)
   <223> CTLA4/Fc
<400> 19
   atg agg acc tgg ccc tgc act ctc ctg ttt ttt ctt ctc ttc atc cct        48
   Met Arg Thr Trp Pro Cys Thr Leu Leu Phe Phe Leu Leu Phe Ile Pro
     1               5                  10                  15
   gtc ttc tgc aaa gca atg cac gtg gcc cag cct gct gtg gta ctg gcc        96
   Val Phe Cys Lys Ala Met His Val Ala Gln Pro Ala Val Val Leu Ala
                20                  25                  30
   agc agc cga ggc atc gcc agc ttt gtg tgt gag tat gca tct cca ggc       144
   Ser Ser Arg Gly Ile Ala Ser Phe Val Cys Glu Tyr Ala Ser Pro Gly
            35                  40                  45
   aaa gcc act gag gtc cgg gtg aca gtg ctt cgg cag gct gac agc cag        192
   Lys Ala Thr Glu Val Arg Val Thr Val Leu Arg Gln Ala Asp Ser Gln
       50                  55                  60
   gtg act gaa gtc tgt gcg gca acc tac atg atg ggg aat gag ttg acc        240
   Val Thr Glu Val Cys Ala Ala Thr Tyr Met Met Gly Asn Glu Leu Thr
   65                  70                  75                  80
   ttc cta gat gat tcc atc tgc acg ggc acc tcc agt gga aat caa gtg        288
   Phe Leu Asp Asp Ser Ile Cys Thr Gly Thr Ser Ser Gly Asn Gln Val
                   85                  90                  95
   aac ctc act atc caa gga ctg agg gcc atg gac acg gga ctc tac atc        336
   Asn Leu Thr Ile Gln Gly Leu Arg Ala Met Asp Thr Gly Leu Tyr Ile
               100                 105                 110
   tgc aag gtg gag ctc atg tac cca ccg cca tac tac ctg ggc ata ggc        384
   Cys Lys Val Glu Leu Met Tyr Pro Pro Pro Tyr Tyr Leu Gly Ile Gly
           115                 120                 125
   aac gga acc cag att tat gta att gat cca gaa ccg tgc cca gat tct        432
   Asn Gly Thr Gln Ile Tyr Val Ile Asp Pro Glu Pro Cys Pro Asp Ser
       130                 135                 140
   gca gag ccc aaa tct tgt gac aaa act cac aca tgc cca ccg tgc cca        480
   Ala Glu Pro Lys Ser Cys Asp Lys Thr His Thr Cys Pro Pro Cys Pro
   145                 150                 155                 160
   gca cct gaa ctc ctg ggg gga ccg tca gtc ttc ctc ttc ccc cca aaa        528
   Ala Pro Glu Leu Leu Gly Gly Pro Ser Val Phe Leu Phe Pro Pro Lys
                   165                 170                 175
   ccc aag gac acc ctc atg atc tcc cgg acc cct gag gtc aca tgc gtg        576
   Pro Lys Asp Thr Leu Met Ile Ser Arg Thr Pro Glu Val Thr Cys Val
               180                 185                 190
   gtg gtg gac gtg agc cac gaa gac cct gag gtc aag ttc aac tgg tac        624
   Val Val Asp Val Ser His Glu Asp Pro Glu Val Lys Phe Asn Trp Tyr
           195                 200                 205
   gtg gac ggc gtg gag gtg cat aat gcc aag aca aag ccg cgg gag gag       672
   Val Asp Gly Val Glu Val His Asn Ala Lys Thr Lys Pro Arg Glu Glu
       210                 215                 220
   cag tac aac agc acg tac cgg gtg gtc agc gtc ctc acc gtc ctg cac       720
   Gln Tyr Asn Ser Thr Tyr Arg Val Val Ser Val Leu Thr Val Leu His
   225                 230                 235                 240
   cag gac tgg ctg aat ggc aag gag tac aag tgc aag gtc tcc aac aaa       768
   Gln Asp Trp Leu Asn Gly Lys Glu Tyr Lys Cys Lys Val Ser Asn Lys
                   245                 250                 255
   gcc ctc cca gcc ccc atc gag aaa acc atc tcc aaa gcc aaa ggg cag       816
   Ala Leu Pro Ala Pro Ile Glu Lys Thr Ile Ser Lys Ala Lys Gly Gln
               260                 265                 270
   ccc cga gaa cca cag gtg tac acc ctg ccc cca tcc cgg gat gag ctg       864
   Pro Arg Glu Pro Gln Val Tyr Thr Leu Pro Pro Ser Arg Asp Glu Leu
           275                 280                 285
   acc aag aac cag gtc agc ctg acc tgc ctg gtc aaa ggc ttc tat ccc       912
   Thr Lys Asn Gln Val Ser Leu Thr Cys Leu Val Lys Gly Phe Tyr Pro
       290                 295                 300
   agc gac atc gcc gtg gag tgg gag agc aat ggg cag ccg gag aac aac       960
   Ser Asp Ile Ala Val Glu Trp Glu Ser Asn Gly Gln Pro Glu Asn Asn
   305                 310                 315                 320
   tac aag acc acg cct ccc gtg ctg gac tcc gac ggc tcc ttc ttc ctc      1008
   Tyr Lys Thr Thr Pro Pro Val Leu Asp Ser Asp Gly Ser Phe Phe Leu
                   325                 330                 335
   tac agc aag ctc acc gtg gac aag agc agg tgg cag cag ggg aac gtc      1056
   Tyr Ser Lys Leu Thr Val Asp Lys Ser Arg Trp Gln Gln Gly Asn Val
               340                 345                 350
   ttc tca tgc tcc gtg atg cat gag gct ctg cac aac cac tac acg cag      1104
   Phe Ser Cys Ser Val Met His Glu Ala Leu His Asn His Tyr Thr Gln
           355                 360                 365
   aag agc ctc tcc ctg tct ccg ggt aaa          tga                     1134
   Lys Ser Leu Ser Leu Ser Pro Gly Lys
       370                 375
<210> 20
   <211> 377
   <212> PRT
   <213> Homo sapiens
<400> 20
   Met Arg Thr Trp Pro Cys Thr Leu Leu Phe Phe Leu Leu Phe Ile Pro
    1               5                   10                  15
   Val Phe Cys Lys Ala Met His Val Ala Gln Pro Ala Val Val Leu Ala
               20                  25                  30
   Ser Ser Arg Gly Ile Ala Ser Phe Val Cys Glu Tyr Ala Ser Pro Gly
           35                  40                  45
   Lys Ala Thr Glu Val Arg Val Thr Val Leu Arg Gln Ala Asp Ser Gln
       50                  55                  60
   Val Thr Glu Val Cys Ala Ala Thr Tyr Met Met Gly Asn Glu Leu Thr
   65                  70                  75                  80
   Phe Leu Asp Asp Ser Ile Cys Thr Gly Thr Ser Ser Gly Asn Gln Val
                   85                  90                  95
   Asn Leu Thr Ile Gln Gly Leu Arg Ala Met Asp Thr Gly Leu Tyr Ile
               100                 105                 110
   Cys Lys Val Glu Leu Met Tyr Pro Pro Pro Tyr Tyr Leu Gly Ile Gly
           115                 120                 125
   Asn Gly Thr Gln Ile Tyr Val Ile Asp Pro Glu Pro Cys Pro Asp Ser
       130                 135                 140
   Ala Glu Pro Lys Ser Cys Asp Lys Thr His Thr Cys Pro Pro Cys Pro
   145 150 155 160
   Ala Pro Glu Leu Leu Gly Gly Pro Ser Val Phe Leu Phe Pro Pro Lys
   165 170 175
   Pro Lys Asp Thr Leu Met Ile Ser Arg Thr Pro Glu Val Thr Cys Val
   180 185 190
   Val Val Asp Val Ser His Glu Asp Pro Glu Val Lys Phe Asn Trp Tyr
   195 200 205
   Val Asp Gly Val Glu Val His Asn Ala Lys Thr Lys Pro Arg Glu Glu
   210 215 220
   Gln Tyr Asn Ser Thr Tyr Arg Val Val Ser Val Leu Thr Val Leu His
   225 230 235 240
   Gln Asp Trp Leu Asn Gly Lys Glu Tyr Lys Cys Lys Val Ser Asn Lys
   245 250 255
   Ala Leu Pro Ala Pro Ile Glu Lys Thr Ile Ser Lys Ala Lys Gly Gln
   260 265 270
   Pro Arg Glu Pro Gln Val Tyr Thr Leu Pro Pro Ser Arg Asp Glu Leu
   275 280 285
   Thr Lys Asn Gln Val Ser Leu Thr Cys Leu Val Lys Gly Phe Tyr Pro
   290 295 300
   Ser Asp Ile Ala Val Glu Trp Glu Ser Asn Gly Gln Pro Glu Asn Asn
   305 310 315 320
   Tyr Lys Thr Thr Pro Pro Val Leu Asp Ser Asp Gly Ser Phe Phe Leu
   325 330 335
   Tyr Ser Lys Leu Thr Val Asp Lys Ser Arg Trp Gln Gln Gly Asn Val
   340 345 350
   Phe Ser Cys Ser Val Met His Glu Ala Leu His Asn His Tyr Thr Gln
   355 360 365
   Lys Ser Leu Ser Leu Ser Pro Gly Lys
   370 375
<210> 21
   <211> 1509
   <212> DNA
   <213> Homo sapiens
<220>
   <221> CDS
   <222> (1)..(1506)
   <223> CTLA4-CTLA4/Fc
<400> 21
   atg agg acc tgg ccc tgc act ctc ctg ttt ttt ctt ctc ttc atc cct        48
   Met Arg Thr Trp Pro Cys Thr Leu Leu Phe Phe Leu Leu Phe Ile Pro
     1               5                  10                  15
   gtc ttc tgc aaa gca atg cac gtg gcc cag cct gct gtg gta ctg gcc        96
   Val Phe Cys Lys Ala Met His Val Ala Gln Pro Ala Val Val Leu Ala
               20                  25                  30
   agc agc cga ggc atc gcc agc ttt gtg tgt gag tat gca tct cca ggc       144
   Ser Ser Arg Gly Ile Ala Ser Phe Val Cys Glu Tyr Ala Ser Pro Gly
           35                  40                  45
   aaa gcc act gag gtc cgg gtg aca gtg ctt cgg cag gct gac agc cag       192
   Lys Ala Thr Glu Val Arg Val Thr Val Leu Arg Gln Ala Asp Ser Gln
       50                  55                  60
   gtg act gaa gtc tgt gcg gca acc tac atg atg ggg aat gag ttg acc       240
   Val Thr Glu Val Cys Ala Ala Thr Tyr Met Met Gly Asn Glu Leu Thr
   65                  70                  75                  80
   ttc cta gat gat tcc atc tgc acg ggc acc tcc agt gga aat caa gtg       288
   Phe Leu Asp Asp Ser Ile Cys Thr Gly Thr Ser Ser Gly Asn Gln Val
                   85                  90                  95
   aac ctc act atc caa gga ctg agg gcc atg gac acg gga ctc tac atc      336
   Asn Leu Thr Ile Gln Gly Leu Arg Ala Met Asp Thr Gly Leu Tyr Ile
               100                 105                 110
   tgc aag gtg gag ctc atg tac cca ccg cca tac tac ctg ggc ata ggc      384
   Cys Lys Val Glu Leu Met Tyr Pro Pro Pro Tyr Tyr Leu Gly Ile Gly
           115                 120                 125
   aac gga acc cag att tat gta att gat cca gaa ccg tgc cca gat tcg      432
   Asn Gly Thr Gln Ile Tyr Val Ile Asp Pro Glu Pro Cys Pro Asp Ser
       130                 135                 140
   gat aac atg cac gtg gcc cag cct gct gtg gta ctg gcc agc agc cga      480
   Asp Asn Met His Val Ala Gln Pro Ala Val Val Leu Ala Ser Ser Arg
   145                 150                 155                 160
   ggc atc gcc agc ttt gtg tgt gag tat gca tct cca ggc aaa gcc act      528
   Gly Ile Ala Ser Phe Val Cys Glu Tyr Ala Ser Pro Gly Lys Ala Thr
                   165                 170                 175
   gag gtc cgg gtg aca gtg ctt cgg cag gct gac agc cag gtg act gaa      576
   Glu Val Arg Val Thr Val Leu Arg Gln Ala Asp Ser Gln Val Thr Glu
               180                 185                 190
   gtc tgt gcg gca acc tac atg atg ggg aat gag ttg acc ttc cta gat      624
   Val Cys Ala Ala Thr Tyr Met Met Gly Asn Glu Leu Thr Phe Leu Asp
           195                 200                 205
   gat tcc atc tgc acg ggc acc tcc agt gga aat caa gtg aac ctc act      672
   Asp Ser Ile Cys Thr Gly Thr Ser Ser Gly Asn Gln Val Asn Leu Thr
       210                 215                 220
   atc caa gga ctg agg gcc atg gac acg gga ctc tac atc tgc aag gtg      720
   Ile Gln Gly Leu Arg Ala Met Asp Thr Gly Leu Tyr Ile Cys Lys Val
   225                 230                 235                 240
   gag ctc atg tac cca ccg cca tac tac ctg ggc ata ggc aac gga acc      768
   Glu Leu Met Tyr Pro Pro Pro Tyr Tyr Leu Gly Ile Gly Asn Gly Thr
                   245                 250                 255
   cag att tat gta att gat cca gaa ccg tgc cca gat tct gca gag ccc        816
   Gln Ile Tyr Val Ile Asp Pro Glu Pro Cys Pro Asp Ser Ala Glu Pro
               260                 265                 270
   aaa tct tgt gac aaa act cac aca tgc cca ccg tgc cca gca cct gaa        864
   Lys Ser Cys Asp Lys Thr His Thr Cys Pro Pro Cys Pro Ala Pro Glu
           275                 280                 285
   ctc ctg ggg gga ccg tca gtc ttc ctc ttc ccc cca aaa ccc aag gac        912
   Leu Leu Gly Gly Pro Ser Val Phe Leu Phe Pro Pro Lys Pro Lys Asp
       290                 295                 300
   acc ctc atg atc tcc cgg acc cct gag gtc aca tgc gtg gtg gtg gac        960
   Thr Leu Met Ile Ser Arg Thr Pro Glu Val Thr Cys Val Val Val Asp
   305                 310                 315                 320
   gtg agc cac gaa gac cct gag gtc aag ttc aac tgg tac gtg gac ggc       1008
   Val Ser His Glu Asp Pro Glu Val Lys Phe Asn Trp Tyr Val Asp Gly
                   325                 330                 335
   gtg gag gtg cat aat gcc aag aca aag ccg cgg gag gag cag tac aac       1056
   Val Glu Val His Asn Ala Lys Thr Lys Pro Arg Glu Glu Gln Tyr Asn
               340                 345                 350
   agc acg tac cgg gtg gtc agc gtc ctc acc gtc tgt cac cag gac tgg       1104
   Ser Thr Tyr Arg Val Val Ser Val Leu Thr Val Cys His Gln Asp Trp
           355                 360                 365
   ctg aat ggc aag gag tac aag tgc aag gtc tcc aac aaa gcc ctc cca       1152
   Leu Asn Gly Lys Glu Tyr Lys Cys Lys Val Ser Asn Lys Ala Leu Pro
       370                 375                 380
   gcc ccc atc gag aaa acc atc tcc aaa gcc aaa ggg cag ccc cga gaa       1200
   Ala Pro Ile Glu Lys Thr Ile Ser Lys Ala Lys Gly Gln Pro Arg Glu
   385                 390                 395                 400
   cca cag gtg tac acc ctg ccc cca tcc cgg gat gag ctg acc aag aac       1248
   Pro Gln Val Tyr Thr Leu Pro Pro Ser Arg Asp Glu Leu Thr Lys Asn
                   405                 410                 415
   cag gtc agc ctg acc tgc ctg gtc aaa ggc ttc tat ccc agc gac atc         1296
   Gln Val Ser Leu Thr Cys Leu Val Lys Gly Phe Tyr Pro Ser Asp Ile
               420                 425                 430
   gcc gtg gag tgg gag agc aat ggg cag ccg gag aac aac tac aag acc         1344
   Ala Val Glu Trp Glu Ser Asn Gly Gln Pro Glu Asn Asn Tyr Lys Thr
           435                 440                 445
   acg cct ccc gtg ctg gac tcc gac ggc tcc ttc ttc ctc tac agc aag         1392
   Thr Pro Pro Val Leu Asp Ser Asp Gly Ser Phe Phe Leu Tyr Ser Lys
       450                 455                 460
   ctc acc gtg gac aag agc agg tgg cag cag ggg aac gtc ttc tca tgc         1440
   Leu Thr Val Asp Lys Ser Arg Trp Gln Gln Gly Asn Val Phe Ser Cys
   465                 470                 475                 480
   tcc gtg atg cat gag gct ctg cac aac cac tac acg cag aag agc ctc         1488
   Ser Val Met His Glu Ala Leu His Asn His Tyr Thr Gln Lys Ser Leu
                   485                 490                 495
   tcc ctg tct ccg ggt aaa     tga                                         1509
   Ser Leu Ser Pro Gly Lys
               500
<210> 22
   <211> 502
   <212> PRT
   <213> Homo sapiens
<400> 22
   Met Arg Thr Trp Pro Cys Thr Leu Leu Phe Phe Leu Leu Phe Ile Pro
   1               5                   10                  15
   Val Phe Cys Lys Ala Met His Val Ala Gln Pro Ala Val Val Leu Ala
               20                  25                  30
   Ser Ser Arg Gly Ile Ala Ser Phe Val Cys Glu Tyr Ala Ser Pro Gly
           35                  40                  45
   Lys Ala Thr Glu Val Arg Val Thr Val Leu Arg Gln Ala Asp Ser Gln
        50                  55                  60
   Val Thr Glu Val Cys Ala Ala Thr Tyr Met Met Gly Asn Glu Leu Thr
   65                  70                  75                  80
   Phe Leu Asp Asp Ser Ile Cys Thr Gly Thr Ser Ser Gly Asn Gln Val
                   85                  90                  95
   Asn Leu Thr Ile Gln Gly Leu Arg Ala Met Asp Thr Gly Leu Tyr Ile
               100                 105                 110
   Cys Lys Val Glu Leu Met Tyr Pro Pro Pro Tyr Tyr Leu Gly Ile Gly
           115                 120                 125
   Asn Gly Thr Gln Ile Tyr Val Ile Asp Pro Glu Pro Cys Pro Asp Ser
       130                 135                 140
   Asp Asn Met His Val Ala Gln Pro Ala Val Val Leu Ala Ser Ser Arg
   145                 150                 155                 160
   Gly Ile Ala Ser Phe Val Cys Glu Tyr Ala Ser Pro Gly Lys Ala Thr
                   165                 170                 175
   Glu Val Arg Val Thr Val Leu Arg Gln Ala Asp Ser Gln Val Thr Glu
               180                 185                 190
   Val Cys Ala Ala Thr Tyr Met Met Gly Asn Glu Leu Thr Phe Leu Asp
           195                 200                 205
   Asp Ser Ile Cys Thr Gly Thr Ser Ser Gly Asn Gln Val Asn Leu Thr
       210                 215                 220
   Ile Gln Gly Leu Arg Ala Met Asp Thr Gly Leu Tyr Ile Cys Lys Val
   225                 230                 235                 240
   Glu Leu Met Tyr Pro Pro Pro Tyr Tyr Leu Gly Ile Gly Asn Gly Thr
                   245                 250                 255
   Gln Ile Tyr Val Ile Asp Pro Glu Pro Cys Pro Asp Ser Ala Glu Pro
               260                 265                 270
   Lys Ser Cys Asp Lys Thr His Thr Cys Pro Pro Cys Pro Ala Pro Glu
           275                 280                 285
   Leu Leu Gly Gly Pro Ser Val Phe Leu Phe Pro Pro Lys Pro Lys Asp
       290                 295                 300
   Thr Leu Met Ile Ser Arg Thr Pro Glu Val Thr Cys Val Val Val Asp
   305                 310                 315                 320
   Val Ser His Glu Asp Pro Glu Val Lys Phe Asn Trp Tyr Val Asp Gly
                   325                 330                 335
   Val Glu Val His Asn Ala Lys Thr Lys Pro Arg Glu Glu Gln Tyr Asn
               340                 345                 350
   Ser Thr Tyr Arg Val Val Ser Val Leu Thr Val Cys His Gln Asp Trp
           355                 360                 365
   Leu Asn Gly Lys Glu Tyr Lys Cys Lys Val Ser Asn Lys Ala Leu Pro
       370                 375                 380
   Ala Pro Ile Glu Lys Thr Ile Ser Lys Ala Lys Gly Gln Pro Arg Glu
   385                 390                 395                 400
   Pro Gln Val Tyr Thr Leu Pro Pro Ser Arg Asp Glu Leu Thr Lys Asn
                   405                 410                 415
   Gln Val Ser Leu Thr Cys Leu Val Lys Gly Phe Tyr Pro Ser Asp Ile
               420                 425                 430
   Ala Val Glu Trp Glu Ser Asn Gly Gln Pro Glu Asn Asn Tyr Lys Thr
           435                 440                 445
   Thr Pro Pro Val Leu Asp Ser Asp Gly Ser Phe Phe Leu Tyr Ser Lys
       450                 455                 460
   Leu Thr Val Asp Lys Ser Arg Trp Gln Gln Gly Asn Val Phe Ser Cys
   465                 470                 475                 480
   Ser Val Met His Glu Ala Leu His Asn His Tyr Thr Gln Lys Ser Leu
                   485                 490                 495
   Ser Leu Ser Pro Gly Lys
               500
<210> 23
   <211> 1335
   <212> DNA
   <213> Homo sapiens
<220>
   <221> CDS
   <222> (1)..(1332)
   <223> TNFR1/FC
<400> 23
   atg ggc ctc tcc acc gtg cct gac ctg ctg ctg ccg ctg gtg ctc ctg        48
   Met Gly Leu Ser Thr Val Pro Asp Leu Leu Leu Pro Leu Val Leu Leu
   1               5                   10                  15
   gag ctg ttg gtg gga ata tac ccc tca ggg gtt att gga ctg gtc cct        96
   Glu Leu Leu Val Gly Ile Tyr Pro Ser Gly Val Ile Gly Leu Val Pro
               20                  25                  30
   cac cta ggg gac agg gag aag aga gat agt gtg tgt ccc caa gga aaa       144
   His Leu Gly Asp Arg Glu Lys Arg Asp Ser Val Cys Pro Gln Gly Lys
           35                  40                  45
   tat atc cac cct caa aat aat tcg att tgc tgt acc aag tgc cac aaa       192
   Tyr Ile His Pro Gln Asn Asn Ser Ile Cys Cys Thr Lys Cys His Lys
       50                  55                  60
   gga acc tac ttg tac aat gac tgt cca ggc ccg ggg cag gat acg gac       240
   Gly Thr Tyr Leu Tyr Asn Asp Cys Pro Gly Pro Gly Gln Asp Thr Asp
   65                  70                  75                  80
   tgc agg gag tgt gag agc ggc tcc ttc acc gct tca gaa aac cac ctc       288
   Cys Arg Glu Cys Glu Ser Gly Ser Phe Thr Ala Ser Glu Asn His Leu
                    85                  90                  95
   aga cac tgc ctc agc tgc tcc aaa tgc cga aag gaa atg ggt cag gtg       336
   Arg His Cys Leu Ser Cys Ser Lys Cys Arg Lys Glu Met Gly Gln Val
               100                 105                 110
   gag atc tct tct tgc aca gtg gac cgg gac acc gtg tgt ggc tgc agg       384
   Glu Ile Ser Ser Cys Thr Val Asp Arg Asp Thr Val Cys Gly Cys Arg
           115                 120                 125
   aag aac cag tac cgg cat tat tgg agt gaa aac ctt ttc cag tgc ttc       432
   Lys Asn Gln Tyr Arg His Tyr Trp Ser Glu Asn Leu Phe Gln Cys Phe
       130                 135                 140
   aat tgc agc ctc tgc ctc aat ggg acc gtg cac ctc tcc tgc cag gag       480
   Asn Cys Ser Leu Cys Leu Asn Gly Thr Val His Leu Ser Cys Gln Glu
   145                 150                 155                 160
   aaa cag aac acc gtg tgc acc tgc cat gca ggt ttc ttt cta aga gaa       528
   Lys Gln Asn Thr Val Cys Thr Cys His Ala Gly Phe Phe Leu Arg Glu
                   165                 170                 175
   aac gag tgt gtc tcc tgt agt aac tgt aag aaa agc ctg gag tgc acg       576
   Asn Glu Cys Val Ser Cys Ser Asn Cys Lys Lys Ser Leu Glu Cys Thr
               180                 185                 190
   aag ttg tgc cta ccc cag att gag aat gtt aag ggc act gag gac tca       624
   Lys Leu Cys Leu Pro Gln Ile Glu Asn Val Lys Gly Thr Glu Asp Ser
           195                 200                 205
   ggc acc aca gca gag ccc aaa tct tgt gac aaa act cac aca tgc cca       672
   Gly Thr Thr Ala Glu Pro Lys Ser Cys Asp Lys Thr His Thr Cys Pro
       210                 215                 220
   ccg tgc cca gca cct gaa ctc ctg ggg gga ccg tca gtc ttc ctc ttc       720
   Pro Cys Pro Ala Pro Glu Leu Leu Gly Gly Pro Ser Val Phe Leu Phe
   225                 230                 235                 240
   ccc cca aaa ccc aag gac acc ctc atg atc tcc cgg acc cct gag gtc      768
   Pro Pro Lys Pro Lys Asp Thr Leu Met Ile Ser Arg Thr Pro Glu Val
                   245                 250                 255
   aca tgc gtg gtg gtg gac gtg agc cac gaa gac cct gag gtc aag ttc      816
   Thr Cys Val Val Val Asp Val Ser His Glu Asp Pro Glu Val Lys Phe
               260                 265                 270
   aac tgg tac gtg gac ggc gtg gag gtg cat aat gcc aag aca aag ccg      864
   Asn Trp Tyr Val Asp Gly Val Glu Val His Asn Ala Lys Thr Lys Pro
           275                 280                 285
   cgg gag gag cag tac aac agc acg tac cgg gtg gtc agc gtc ctc acc      912
   Arg Glu Glu Gln Tyr Asn Ser Thr Tyr Arg Val Val Ser Val Leu Thr
       290                 295                 300
   gtc ctg cac cag gac tgg ctg aat ggc aag gag tac aag tgc aag gtc      960
   Val Leu His Gln Asp Trp Leu Asn Gly Lys Glu Tyr Lys Cys Lys Val
   305                 310                 315                 320
   tcc aac aaa gcc ctc cca gcc ccc atc gag aaa acc atc tcc aaa gcc     1008
   Ser Asn Lys Ala Leu Pro Ala Pro Ile Glu Lys Thr Ile Ser Lys Ala
                   325                 330                 335
   aaa ggg cag ccc cga gaa cca cag gtg tac acc ctg ccc cca tcc cgg     1056
   Lys Gly Gln Pro Arg Glu Pro Gln Val Tyr Thr Leu Pro Pro Ser Arg
               340                 345                 350
   gat gag ctg acc aag aac cag gtc agc ctg acc tgc ctg gtc aaa ggc     1104
   Asp Glu Leu Thr Lys Asn Gln Val Ser Leu Thr Cys Leu Val Lys Gly
           355                 360                 365
   ttc tat ccc agc gac atc gcc gtg gag tgg gag agc aat ggg cag ccg     1152
   Phe Tyr Pro Ser Asp Ile Ala Val Glu Trp Glu Ser Asn Gly Gln Pro
       370                 375                 380
   gag aac aac tac aag acc acg cct ccc gtg ctg gac tcc gac ggc tcc     1200
   Glu Asn Asn Tyr Lys Thr Thr Pro Pro Val Leu Asp Ser Asp Gly Ser
   385                 390                 395                 400
   tcc ttc ctc tac agc aag ctc acc gtg gac aag agc agg tgg cag cag         1248
   Ser Phe Leu Tyr Ser Lys Leu Thr Val Asp Lys Ser Arg Trp Gln Gln
                   405                 410                 415
   ggg aac gtc ttc tca tgc tcc gtg atg cat gag gct ctg cac aac cac         1296
   Gly Asn Val Phe Ser Cys Ser Val Met His Glu Ala Leu His Asn His
               420                 425                 430
   tac acg cag aag agc ctc tcc ctg tct ccg ggt aaa          tga            1335
   Tyr Thr Gln Lys Ser Leu Ser Leu Ser Pro Gly Lys
           435                 440
<210> 24
   <211> 444
   <212> PRT
   <213> Homo sapiens
<400> 24
   Met Gly Leu Ser Thr Val Pro Asp Leu Leu Leu Pro Leu Val Leu Leu
    1               5                  10                  15
   Glu Leu Leu Val Gly Ile Tyr Pro Ser Gly Val Ile Gly Leu Val Pro
               20                  25                  30
   His Leu Gly Asp Arg Glu Lys Arg Asp Ser Val Cys Pro Gln Gly Lys
           35                  40                  45
   Tyr Ile His Pro Gln Asn Asn Ser Ile Cys Cys Thr Lys Cys His Lys
       50                  55                  60
   Gly Thr Tyr Leu Tyr Asn Asp Cys Pro Gly Pro Gly Gln Asp Thr Asp
   65                  70                  75                  80
   Cys Arg Glu Cys Glu Ser Gly Ser Phe Thr Ala Ser Glu Asn His Leu
                   85                  90                  95
   Arg His Cys Leu Ser Cys Ser Lys Cys Arg Lys Glu Met Gly Gln Val
               100                 105                 110
   Glu Ile Ser Ser Cys Thr Val Asp Arg Asp Thr Val Cys Gly Cys Arg
   115 120 125
   Lys Asn Gln Tyr Arg His Tyr Trp Ser Glu Asn Leu Phe Gln Cys Phe
   130 135 140
   Asn Cys Ser Leu Cys Leu Asn Gly Thr Val His Leu Ser Cys Gln Glu
   145 150 155 160
   Lys Gln Asn Thr Val Cys Thr Cys His Ala Gly Phe Phe Leu Arg Glu
   165 170 175
   Asn Glu Cys Val Ser Cys Ser Asn Cys Lys Lys Ser Leu Glu Cys Thr
   180 185 190
   Lys Leu Cys Leu Pro Gln Ile Glu Asn Val Lys Gly Thr Glu Asp Ser
   195 200 205
   Gly Thr Thr Ala Glu Pro Lys Ser Cys Asp Lys Thr His Thr Cys Pro
   210 215 220
   Pro Cys Pro Ala Pro Glu Leu Leu Gly Gly Pro Ser Val Phe Leu Phe
   225 230 235 240
   Pro Pro Lys Pro Lys Asp Thr Leu Met Ile Ser Arg Thr Pro Glu Val
   245 250 255
   Thr Cys Val Val Val Asp Val Ser His Glu Asp Pro Glu Val Lys Phe
   260 265 270
   Asn Trp Tyr Val Asp Gly Val Glu Val His Asn Ala Lys Thr Lys Pro
   275 280 285
   Arg Glu Glu Gln Tyr Asn Ser Thr Tyr Arg Val Val Ser Val Leu Thr
   290 295 300
   Val Leu His Gln Asp Trp Leu Asn Gly Lys Glu Tyr Lys Cys Lys Val
   305 310 315 320
   Ser Asn Lys Ala Leu Pro Ala Pro Ile Glu Lys Thr Ile Ser Lys Ala
                   325                 330                 335
   Lys Gly Gln Pro Arg Glu Pro Gln Val Tyr Thr Leu Pro Pro Ser Arg
               340                 345                 350
   Asp Glu Leu Thr Lys Asn Gln Val Ser Leu Thr Cys Leu Val Lys Gly
           355                 360                 365
   Phe Tyr Pro Ser Asp Ile Ala Val Glu Trp Glu Ser Asn Gly Gln Pro
       370                 375                 380
   Glu Asn Asn Tyr Lys Thr Thr Pro Pro Val Leu Asp Ser Asp Gly Ser
   385                 390                 395                 400
   Ser Phe Leu Tyr Ser Lys Leu Thr Val Asp Lys Ser Arg Trp Gln Gln
                   405                 410                 415
   Gly Asn Val Phe Ser Cys Ser Val Met His Glu Ala Leu His Asn His
               420                 425                 430
   Tyr Thr Gln Lys Ser Leu Ser Leu Ser Pro Gly Lys
           435                 440
<210> 25
   <211> 2028
   <212> DNA
   <213> Homo sapiens
<220>
   <221> CDS
   <222> (1)..(2025)
   <223> TNFR2-TNFR1/Fc
<400> 25
   atg gcg ccc gtc gcc gtc tgg gcc gcg ctg gcc gtc gga ctg gag ctc        48
   tgg gct gcg gcg cac gcc ttg ccc gcc cag gtg gca ttt aca ccc tac      96
   gcc ccg gag ccc ggg agc aca tgc cgg ctc aga gaa tac tat gac cag     144
   aca gct cag atg tgc tgc agc aaa tgc tcg ccg ggc caa cat gca aaa     192
   gtc ttc tgt acc aag acc tcg gac acc gtg tgt gac tcc tgt gag gac     240
   agc aca tac acc cag ctc tgg aac tgg gtt ccc gag tgc ttg agc tgt     288
   ggc tcc cgc tgt agc tct gac cag gtg gaa act caa gcc tgc act cgg     336
   gaa cag aac cgc atc tgc acc tgc agg ccc ggc tgg tac tgc gcg ctg     384
   agc aag cag gag ggg tgc cgg ctg tgc gcg ccg ctg cgc aag tgc cgc     432
   ccg ggc ttc ggc gtg gcc aga cca gga act gaa aca tca gac gtg gtg     480
   tgc aag ccc tgt gcc ccg ggg acg ttc tcc aac acg act tca tcc acg     528
   gat att tgc agg ccc cac cag atc tgt aac gtg gtg gcc atc cct ggg      576
   aat gca agc atg gat gca gtc tgc acg tcc acg tcc ccc acc cgg agt      624
   atg gcc cca ggg gca gta cac tta ccc cag cca gtg tcc aca cga tcc      672
   caa cac acg cag cca act cca gaa ccc agc act gct cca agc acc tcc      720
   ttc ctg ctc cca atg ggc ccc agc ccc cca gct gaa ggg agc gga tcc      768
   ggg aac att tca ctg gtc cct cac cta ggg gac agg gag aag aga gat      816
   agt gtg tgt ccc caa gga aaa tat atc cac cct caa aat aat tcg att      864
   tgc tgt acc aag tgc cac aaa gga acc tac ttg tac aat gac tgt cca      912
   ggc ccg ggg cag gat acg gac tgc agg gag tgt gag agc ggc tcc ttc      960
   acc gct tca gaa aac cac ctc aga cac tgc ctc agc tgc tcc aaa tgc     1008
   cga aag gaa atg ggt cag gtg gag atc tct tct tgc aca gtg gac cgg        1056
   gac acc gtg tgt ggc tgc agg aag aac cag tac cgg cat tat tgg agt        1104
   gaa aac ctt ttc cag tgc ttc aat tgc agc ctc tgc ctc aat ggg acc        1152
   gtg cac ctc tcc tgc cag gag aaa cag aac acc gtg tgc acc tgc cat        1200
   gca ggt ttc ttt cta aga gaa aac gag tgt gtc tcc tgt agt aac tgt        1248
   aag aaa agc ctg gag tgc acg aag ttg tgc cta ccc cag att gag aat        1296
   gtt aag ggc act gag gac tca ggc acc aca gca gag ccc aaa tct tgt        1344
   gac aaa act cac aca tgc cca ccg tgc cca gca cct gaa ctc ctg ggg        1392
   gga ccg tca gtc ttc ctc ttc ccc cca aaa ccc aag gac acc ctc atg        1440
   atc tcc cgg acc cct gag gtc aca tgc gtg gtg gtg gac gtg agc cac        1488
   gaa gac cct gag gtc aag ttc aac tgg tac gtg gac ggc gtg gag gtg      1536
   cat aat gcc aag aca aag ccg cgg gag gag cag tac aac agc acg tac      1584
   cgg gtg gtc agc gtc ctc acc gtc ctg cac cag gac tgg ctg aat ggc      1632
   aag gag tac aag tgc aag gtc tcc aac aaa gcc ctc cca gcc ccc atc      1680
   gag aaa acc atc tcc aaa gcc aaa ggg cag ccc cga gaa cca cag gtg      1728
   tac acc ctg ccc cca tcc cgg gat gag ctg acc aag aac cag gtc agc      1776
   ctg acc tgc ctg gtc aaa ggc ttc tat ccc agc gac atc gcc gtg gag      1824
   tgg gag agc aat ggg cag ccg gag aac aac tac aag acc acg cct ccc      1872
   gtg ctg gac tcc gac ggc tcc tcc ttc ctc tac agc aag ctc acc gtg      1920
   gac aag agc agg tgg cag cag ggg aac gtc ttc tca tgc tcc gtg atg      1968
   cat gag gct ctg cac aac cac tac acg cag aag agc ctc tcc ctg tct        2016
   ccg ggt aaa    tga                                                     2028
<210> 26
   <211> 675
   <212> PRT
   <213> Homo sapiens
<400> 26

## Description

### FIELD OF THE INVENTION

The invention relates to the usage of organic substances, particularly lactic acid obtained by microbiological fermentation of cereal starch and esters of said acid, for production of biodegradable lactic acid polymers, and the usage of the biodegradable polymers produced in this way. The invention also relates to food processing and chemical technology. The application areas of the invention are the production of starch, phytoproteins, lactic acid and its derivatives, including its salts, esters and biodegradable polymers (PLA).

### STATE OF ART

Plastics with long decomposition period are a major source of environmental pollution. The main part of such pollution comes from products that are designed for one-time consumption and have a short consumption cycle, which so far have mostly been made from oil plastics that are not easily decomposed in nature. The reduction of pollution by recycling use is mostly economically non-expedient and often impossible in terms of hygiene. A real solution would be using biodegradable polymers, which adjustable decomposition period. One of the most effective materials of this type is the lactic acid polymer, i.e. polylactate, which until now has been used primarily in medical products, but not for consumer goods.

A well-known method for producing the lactic acid for its polymers is microbiological synthesis that uses lactic acid fermentation of pre-processed starch with lactic acid bacteria such as *Lactobacillus amylophilus* and *L. amylophorus* or *Lactobacillus bulgaricum* and *L. delbrueckii.* The raw materials starch-rich, crops have been used, whereas corn and manioc (cassava) being the best respectively among cereals and tuber cultures. Separation of starch from and its purification from the crop and tubers is technologically known. Since most of the lactic acid bacteria cannot utilize starch as a growth substrate, it must be converted into sugar (glucose) in advance using appropriate enzymes, which is performed in the following order: starch → dextrin → maltose → glucose, and after that the fermentation process is initiated. The lactic acid produced through fermentation is separated from the solution either in the form of calcium salts by sedimentation, by extraction with anon-polar solvents, or electrodialysis, and if necessary, purified using esterification and selective membranes (WO9950345, US4,885,247, EP0657542A1, US5,053,485, US5,503,750, US5,723,639, CN1050203, JP4359014 and WO9509879). Residues of the fractionation and fermentation processes of grain can be used as forage additives.

In connection with the development of the corresponding technology, lactic acid polyester that has been used mostly in exclusive medical devices is turning into a base material of consumer plastics. The most common name of lactic acid polyester in relation with its production technique over lactide is polylactic acid (PLA) (Witzke, D.R.; Narayan, R., Kolstad, J.J. Macromolecules, 1997, 30, 7075). Optically pure lactic acid is preferred for PLA synthesis, and the sole industrial manufacturing technique of said lactic acid is the microbiological production from sugars and their blends. Considering the volumes of lactic acid necessary for economically expedient production of PLA, the main raw material available in the necessary quantity is starch, particularly cereal starch.

Considering the aforesaid, a common production line from cereal to PLA plastic is being designed. So far, the technology of production of PLA is divided into three separate plants: one for production of fermentable sugars, another for fermentation of sugars into lactic acid, and the third for PLA synthesis. A common, integral technological production scheme for producing PLA from cereal has not been patented yet, and the separateness of the production lines does not enable co-ordinated use of the resources, primarily in terms of water, energy, and mineral and nitrogen compounds found in cereal.

According to the most common cereal processing schemes, the fibre-rich outer layer of grains is removed at first - fractionation of bran. Upon further processing of cereal, fractions of starch and protein are separated. Such plants have been founded already since 1835 (Godon et al., Qual. Plant Foods Hum. Nutr., 1983 33:161). So far, water has been used upon fractionation (Fellers, In Industrial Uses of Cereals, Ed. Y. Pomeranz, American Association of Cereal Chemists, St. Paul, Minn., 1963 207), although an alternative method that includes ethanol as the separation medium has been patented (US5,851,301).

The problem in connection with starch plants is large amount of biogene-rich wastewater generated in the course of fractionation (up to 4 m³ per one ton of starch), which must be separated for producing marketable products (starch, gluten, forage), and the solute biogenes have to be extracted in order to reduce environmental pollution. Usually, wastewater is evaporated and dry biogenes are added for example, to the forage fraction. Evaporation of the water consumes a lot of energy.

Industrial fermentation of lactic acid has been practiced for over a century. The first lactic acid plant where sugars were fermented into lactic acid was founded in 1883 in the USA (Avery Lactate Company, Littleton, Massachusetts). To make feed that is suitable for lactic acid fermentation, sugar and sugar manufacturing residues (molasses), milk whey and partially or fully saccharinized starch have been used. In industry, the most commonly used producer strains come from the genus *Lactobacillus,* of which the most widely used strain for producing lactic acid is *L. delbrueckii* NCIB 8130. The production of optically pure L(+) or D(-) lactic acid for synthesising PLA requires a high level of microbiological purity, because in addition to the general threat of microbial contamination, even other producers of lactic acid may impair the optical purity of lactic acid. The growth temperature of the most commonly used industrial lactic acid bacteria is 42-45 °C, which does not preclude from the threat of microbial contamination. Alternative producers of lactic acid include the strains of the *Bacillus* family (US5,079,164; Payot T. et al. Enzyme and Microbial Technology 24:191-199, 1999; EP0770684; Ohara H. Yahata M. Journal of Fermentation and Bioengineering 1996, Vol 81 Iss 3 pp 272-274) or the lactic acid is produced in the open sytem without sterilisation, by adding lactobacillus belonging to genus *Streptococcus, Lactobacillus* or the like to the organic waste JP2000 245491A,).

Lactic acid is marketed in the form of an 80-percent aqueous solution or to a lesser extent, in the form of salts. Since in the plants that produce lactic acid, the final concentration of lactic acid in the fermentation medium is usually not higher than 10-12%, the production of lactic acid requires a lot of water and energy. In order to make a marketable product, 80-100% of the water involved in the process has to be separated.

Unification of a starch plant and production of lactic acid, which our invention involves, enables a rational use of wastewater that is generated upon fractionation as well as its biogenes for producing the growth medium upon the production of lactic acid and the use of water generated upon concentration of lactic acid for fractionation of cereal. So far, the schemes of production of starch and lactic acid in physically separated plants have not been able to use such an opportunity (US5,453,365, US5,766,439, US6,087,532,

US6,187,951, WO9524498, WO9828433 and EP0614983).

Different solutions of fractionation of grains have been described in the following patents: US4,132,566, US4,217,414, US4,494,530, US4,689,409, US5,439,526 and US5,851,301.

Formation of PLA by polycondensation of lactic acid has already been described in 1932 (Carothers, W. et al. J.Am. Chem. Soc., 1932, vol.54,761). Since the extension of the PLA chains during the polycondensation is difficult to control, most of the patented schemes set forth synthesis of PLA over lactide, using the so-called "*ring-opening*" reaction. There is no large-scale industrial manufacturing of PLA yet. High temperature (up to 200 °C) is needed for lactide production and PLA synthesis. Therefore, unification of a PLA production plant with starch and lactic acid plants enables the use of heat generated in the production of PLA for the liquefaction and saccharification of starch.

Patents US5,723,639, EP0614983, CN1102180 and GB346486 describe the purification of lactate over esters of lactic acid.

The most widely used scheme of PLA synthesis requires synthesis of pure and anhydrous lactide as an intermediate. Several production methods have been described, including using vaporised lactic acid, synthesis from pre-polymer, synthesis from PEG-solution or synthesis from salts (US5,332,839, US5,453,365, US6,005,067; Kamm et al., Acta Biotechnol. 20 (2000) 3-4, 289-304).

Upon usage of a lactate, its salts and esters as feedstock of a polymer, the suitable solutions include those that enable a continuous process (US6,005,067; US5,453,365).

Several methods are suitable for the polymerisation of PLA, as described in patents US6,207,792, US5,543,494, or in articles by Jacobsen et al. Polymer engineering and science, 1999, Vol. 39, N7, 1311-1319, and Schmack G et al. J. of Biotechnol. 86 (2001) 151-160.

Hereinafter we will show that integration of a starch plant, production of lactic acid and its derivatives, and PLA-synthesis into a single production line is both economically and ecologically a new quality, which enables one to produce more rationally and reduce duplicated vaporisation of large amounts of water, preserves raw material and energy sources.

### BACKGROUND OF THE INVENTION

The aim of this invention is to develop a method of production of lactic acid polymers based on the starch received from cereal cultures of the temperate climate zone. These cereal cultures include wheat, rye, triticale, barley and corn, the grains of which contain over 60% starch. The goal is to accelerate the cultivation cycle, i.e. to shorten the current cultivation period of 6-7 days to 2-2.5 days, as well as increasing the optimal temperature of cultivation from the current 44 °C to 52-62 °C, which ensures sterility against almost any contaminating microflora. In addition, the method must ensure upon cultivation a lactic acid yield of not less than 85-90% of the theoretical value, high affinity of the producer to fermented sugar in order to ensure a low concentration of residual sugar, easy separability of microbial cells from the cultivation medium, and high prototrophy to reduce dependency on several growth factors. The given requirements can be fulfilled by using thermotolerant and highly productive bacteria.

The invention concerns an integral solution for the production of lactic acid, lactic acid esters, lactic acid salts and plastics based on lactic acid from cereal starch. In single stages of the integral production scheme, the solutions used are usually current, those realised in production and/or described in patents. The novelty of the invention is achieved by the unification of existing technological entities into one original method, consiting the steps of fractionation, iquefication and saccharification, prefermentation and fermentation, purification, synthesis of ethers and glycosis and polymerisation of polylactic acid (PLA). While the integrated production cycle enables to save natural resources both in terms of water and energy and reduces share of biogenes in wastewater compared to production of starch, lactic acid and PLA in independent plants - thus, it simplifies abiding by the environmental regulations.

### DESCRIPTION OF THE TECHNOLOGICAL PROCESS

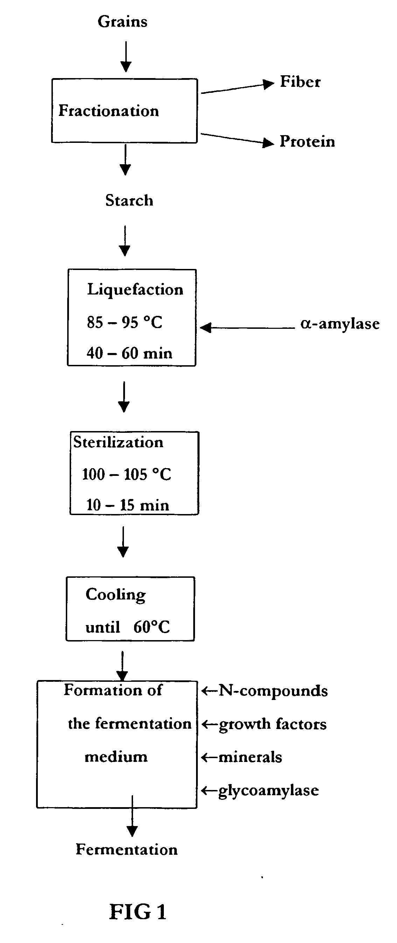

Upon wet milling and fractionation of grains, a starch fraction is produced, from which the most of the fiber and a part of proteins as the by-products are removed. Using hydrocyclones enables one to separate from the produced starch its purer fraction, the so-called A-starch, which can be marketed as a separate product. In order to produce lactic acid polymers, it is possible to use the purer A-starch as well as so-called B-starch, which contains more additives. In the latter case, the minerals and nitrogen compounds can be marketed as growth factors. Thereafter, starch is liquefied at 75-90 °C, then sterilized or pasteurised and used as a fermentation medium to which growth factors are added. In this stage, polysaccharides (i.e. starch) are decomposed into monosaccharides (glucose) using appropriate enzymes and then the produced sugars are fermented into lactic acid (CH₃-CH(OH)-COOH) using corresponding bacteria. This process is schematically described in Figure 1. Upon the liquefaction and saccharification of starch, the water generated in fractionation of grains is used.

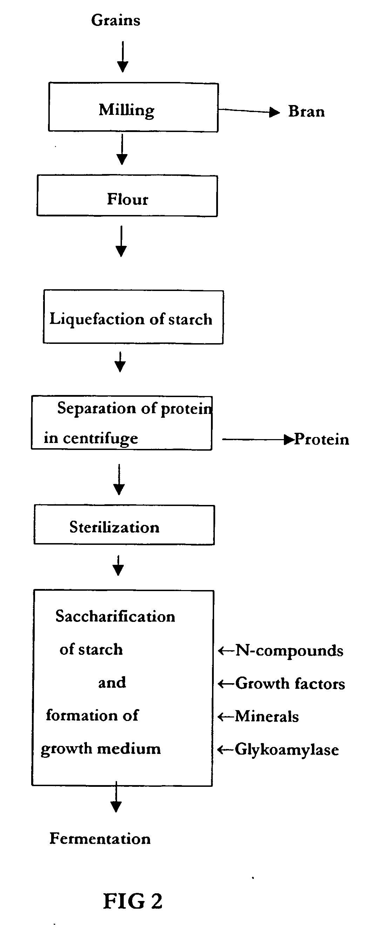

In Figure 2, an alternative opportunity is described in the event that wet milling of grains and the consequent separation of starch from protein is inexpedient. Pursuant to the latter scheme, it is possible to use 70-75% of the starch of the grains for preparation of the fermentation medium. Fibers are separated from the hydrolysate using centrifugal separation. The supernatant is sterilized and used for producing the fermentation medium, adding yeast extract as a growth factor.

Usually, lactic acid producers require a complex set of growth factors, the exact composition of which has never been described in a uniform way in connection with the strains used. The requirements of the producer used in this patent application in connection with the growth factors are quite completely met by the factors present in the wort and yeast extracts (WO 02/074934). The yeast extract is partially replaceable (up to 75%) with hydrolysate of cereal protein without causing substantial deviations in the production and final concentration of lactate. Substitution of the expensive yeast extract with a cheaper phytoprotein is an opportunity to reduce the production cost of lactic acid. Unification of fractionation and fermentation of grains into one technological scheme also enables the use of cereal protein rationally, including the protein, which remains in the fractionation water of starch.

The lactic acid formation speed upon fermentation depends both on the pH of the medium, and actual concentration of lactic acid. The producers used in this patent application enable the fermentation to produce lactate with the concentration of up to 10% upon stabilisation of pH between 5.5 - 6.0 using CaCO₃ as a neutraliser, but upon application of a relatively long fermentation cycle (up to 120 h). If pH is stabilised between 6.2 - 6.3 (for example, using NH3), the fermentation cycle can be reduced to 50-60 hours, while the final concentration of lactate remains between 7.5 - 12%.

To increase the efficiency of fermentation, the crucial factor is binding the productivity (lactate formation rate per hour per litre) to the final concentration of lactate, which is technologically adjustable by the concentration and composition of growth factors, and pH.

The fermentation process of this patent application is characterised by following parameters:

| | |
|---|---|
| 1. Temperature of fermentation | 52-62 °C |
| 2. Yield of lactate of the glucose used | up to 98% |
| 3. Final concentration of lactate | 8-12% |
| 4. Duration of the cultivation in the batch culture | |
| depending on the pH and the neutraliser | 50 to 120 hours |
| 5. Productivity at lactate concentration of 30-400 mM | up to 4 g·l⁻¹·h⁻¹ |
| 6. Productivity of the fermentation cycle | 0.7-1.5 g·l⁻¹·h⁻¹ |

To ensure efficiency of fermentation, a low content of residual sugars in the medium is crucial. The producers used in this application enable residue-free utilisation of glucose at final concentration of lactate at 8-12%. High temperature of fermentation and the possibility of pH adjustment between 5.5-6.3 enable saccharification to be united with fermentation, preserving suitable conditions for both processes and minimising microbiological contamination.

The separation of lactic acid from the fermentation medium can be carried out using any methods that have been used so far, however preferably using a process which enables continuous technology, for example over esters or salts. The obtained lactic acid is directed to the polymerisation stage of lactic acid. Since lactic acid (2-hydroxypropionic acid) contains both the hydroxyl group as well as carboxyl group, it is a typical monomer used in polymerisation and it can form polymeric chains - polyesters. As a result of the process, polylactic acid is formed.

In principle, polymerisation of lactic acid can be achieved in two ways:
1) Direct polymerisation of lactic acid;
2) Polymerisation of lactic acid from its cyclic dimer - lactide.

The molecular mass of polylactic acid produced upon direct polymerisation of lactic acid is low (3,000-4,000) since under the conditions the reaction polymerisation is in balance with depolymerisation. However, the cyclical dimer of lactic acid, i.e. lactide (3,6-dimethyl-1,4-dioxan-2,5-dion) enables the production of (co)polymers that have a high molecular mass.

### Synthesis of Lactide

To carry out the synthesis of lactide, a presence of the catalyst is necessary. Metals of the groups 4, 5, 8, 9, 10, 14 and 15 of the periodic system or their compounds, tin and tin compounds, zinc oxide, and salts of alkali metals (except sodium) have been used. The series of activity of metal catalysts is the following: Sn > Zn > Zr > Ti > Al. The reaction is carried out at a higher temperature (150-240 °C) in a vacuum by distilling off the forming lactide (boiling point of mesolactide is 142 °C/8 mmHg, melting point 116-119 °C).

### Polymerisation of Lactide

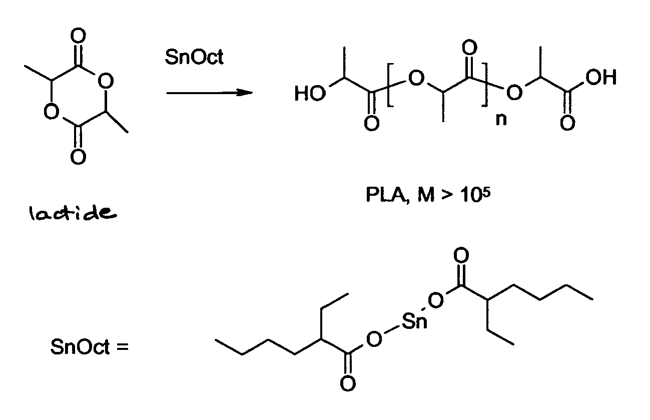

The polymerisation of lactide is performed at a high temperature (~150-250 °C) in the presence of catalysts like SnCl₄, tetraphenyl tin and Sn-2-ethylhexanoate (SnOct), different metal alkoxides like magnesium ethoxide, aluminium isopropoxide, zinc or tin butoxide, zirconium propoxide, tin dibutyl- or tributyl metoxide. The most commonly used catalyst is nevertheless Sn-2-ethylhexanoate (SnOct), a fast polymerisation catalyst, which does not cause substantial racemisation at high temperatures. Polymerisation driven by this catalyst occurs through a non-ionic insertion mechanism.

Optimal concentration of the catalyst depends on the polymerisation temperature and time as well as melting point of the obtained polymer. In the presence of water, high molecular weight polylactic acid is thermodynamically unstable and depolymerisation takes place.

By varying the conditions of polymerisation, it is possible to synthesise polylactic acid with different properties (e.g. change the glass transition temperature of the polymer, synthesise amorphous, semicrystalline or crystalline, straight- or branched-chain polymers).

It is also possible to add other monomers and polymers in the course of polymerisation as well as obtain copolymers. Polymerisation reaction is of first order both in relation to the monomer and catalyst.

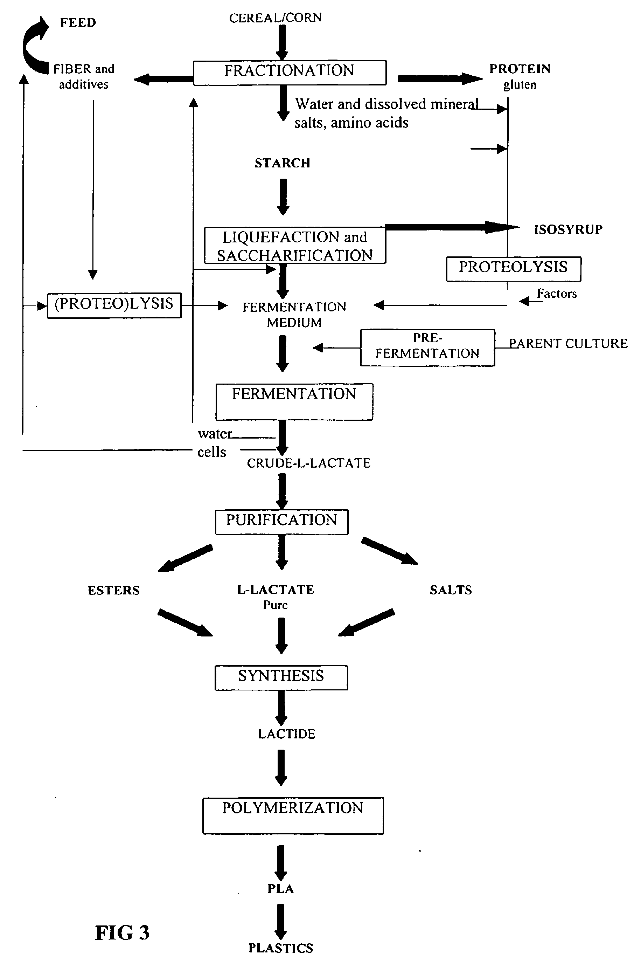

Unification of a starch plant and production of lactic acid as indicated in Figure 3 enables rational utilisation of residual water generated upon fractionation of starch and its biogenes for producing growth medium for production of lactic acid, and the water released upon concentration of lactic acid for fractionation of cereal meal. Current production schemes of starch and lactic acid in physically separate plants cannot use this opportunity. Unification of production of lactic acid with a starch plant enables one to abandon the final drying of starch and protein fractions, which makes starch and protein cheaper than upon purchase from starch production plants.

Unification of fractionation of cereal with fermentation and purification of lactate and polymerisation enables the usage of generated water for fractionation of grains. For fractionation purposes, in principle, any earlier patented solution (referred to above), which enables the obtention of pure A-strach and protein, by separating fraction of fibers from them, is suitable.

In our scheme, liquefaction and saccharification of starch is preferably performed enzymatically, but acid hydrolysis cannot be excluded. Lysis of proteins and peptides is preferably performed with proteases.

Saccharification and proteolysis can occur either in special reactors or be partially or fully performed in fermentation tanks.

Fermentation is preferably performed in a semi-continuous process where the establishment and pre-fermentation of mother culture is a continuous process, but main fermentation is performed in batch fermenters. The obtained lactic acid is neutralised using a suitable base (Ca²⁺, NH⁴⁺, Na⁺, K⁺) or organic bases.

Usable producers include microorganisms that produce pure *L*(+) -or *D*(-)-stereoisomers of lactic acid, but the preferred strains are those producing *L*(+)-lactic acid since *L*-lactate and the products derived thereof have wider field of applications. Of the latter, the strains of the genus *Bacillus* with high temperature tolerance and low need for growth factors are preferable.

Usage of temperature-tolerant strains which growth optimum exceeds 55 °C enables one to abandon the usual sterilization of fermentation equipment by high-pressure steam. Of such producers of lactic acid, the most preferred are microaerophile bacilli that provide more cell mass in the pre-fermentation in microaerophile conditions and thus, increase the productivity of main fermentation. Substitution of lactobacilli with oxygen-tolerant bacilli enables one to control the number of cells in the mother culture better, using microaerophile pre-growth.

Upon separation of lactate from the fermentation medium all existing technological solutions, which are adjustable into an integral production scheme with repetitive use of water, such as evaporation, pervaporation, electrodialysis, and reverse osmosis, are usable. The maximum effect is achieved by technologies that involve concentration of lactic acid by evaporation.

To obtain lactate that is sufficiently pure for polymer synthesis, our scheme prefers purification over lactic acid esters.

The most widely used scheme of synthesis of PLA requires synthesis of pure anhydrous lactide as an intermediate. Several methods have been utilized for obtaining it, like formation from evaporised lactic acid, synthesis from prepolymer, synthesis from PEG-solution or synthesis from salts.

Upon the usage of lactate, its salts as intermediate products and esters as the source materials of polymer, the suitable solutions are those including a continuous process.

Several of the methods described above are suitable for polymerisation of PLA.

The scheme of integration of production presented in this application contains several advantages compared to a separately operating starch plant, lactic acid and its derivatives plant and PLA polymer plant.

### EXAMPLES OF IMPLEMENTATION

### Example 1

Wheat flour was suspended respectively in distilled water and in the water released upon concentration of the fermentation medium (the distillate of the medium used for homofermentative fermentation of lactic acid with *Bacillus coagulans*). The suspensions were allowed to sediment, which resulted in clearly recognisable gluten zone (upper) and fractions of starch and fibers. In two series of experiments, microscopic analysis of the fractions did not indicate any differences.

### Example 2

### Fermentative hydrolysis of starch in the environment of the water utilized for fractionation of cereal.

The water utilized for fractionation was considered to be the water which was obtained from suspending cereal (wheat) flour in water for 20 minutes and from which fiber, starch grains and protein particles were separated by centrifugation at 2,000 g for 15 minutes. Utilising said water, suspension of starch was prepared, the full hydrolysis of which would yield an 800 mM aqueous solution of glucose. Distilled water was used as a control solution. Starch (200 g) was liquefied with industrial enzyme alpha-amylase (Nervanase BT) and saccharinized with glucoamylase (Rakvere Distillery, Estonia). To liquefy starch with alpha-amylase, the temperature was increased to 80 °C in 40 minutes and incubated at this temperature for 10 minutes. Independent of the utilized water, a liquefied dextrose aqueous solution with DE = 17-20 was obtained. The liquefied starch solution was then cooled down to 58 °C and glucoamylase was added. It was incubated at this temperature depending on the amount of glucoamylase added for 3-10 hours up to DE = 97 - 98. The duration of saccharification did not depend on the water utilized. The aforesaid hydrolysate was used to make the feed for the producer of lactic acid.

### Example 3

### Fermentative hydrolysis of gluten in the water used for fractionation of cereal

The water utilized for fractionation was considered to be the water which was obtained from suspending cereal (wheat) flour in water for 20 minutes and from which fiber, starch grains and protein particles were separated by centrifugation at 2,000 g for 15 minutes. Then, a 12-percent aqueous solution of gluten was prepared using fractionation water or distilled water for that purpose. In both cases, the pH was adjusted to 6 and the suspension incubated at 47 °C. For hydrolysis of gluten, an industrial protease complex 'Flavourzyme' (NovoNordisk, Denmark) was used at 50 units per gram of protein. Independent of the utilized water, 33% of the possible theoretical yield (determined by ninhydrin) was released as free amino acids after 30 minutes of exposition. Qualitative analysis of amino acids using thin layer chromatography did not indicate any differences in the amino acid composition of the lysate. The aforesaid hydrolysate was used to make the feed for the producer of lactic acid.

### Example 4

### Fermentation of lactate upon wholemeal feed

The starch of the wholemeal flour suspension was hydrolysed to glucose (15.2%), using enzymological processing with alpha-amylase and glycoamylase. Using Novo-Nordisk Flavourzyme (5 u/g, 50 °C, 20") for that purpose, the grain proteins were converted into a blend of amino acids to the extent of 30% in the initial stage of liquefaction of starch. According to the ninhydrine reaction, the total content of amino acids was 1.2%. The obtained feed was clarified using centrifugation and the supernatant was fermented with *Bacillus coagulans.* 96% of the fermented glucose was converted into *L*-lactate, which accumulates in the fermentation medium as ammonium lactate, resulting in the final concentration of lactic acid of 9.9%. Additional sources of mineral substances and nitrogen compounds were not used.

Cultivation was carried out in anaerobic conditions by mixing at 60 rpm, 57 °C, pH value in the stationary regime at 6.3, NH⁴⁺ was used as a neutraliser. The duration of the cultivation cycle was 42 hours. After the end of fermentation, the cells were centrifuged out and the ammonium lactate in the supernatant was concentrated by distillation to 80%. The distilled water was condensed and collected for following experiments.

### Example 5

### Gluten as the source of N-compounds and growth factors

a) In the proof test, the feed used as the fermentation medium, was composed by adding 800 mM glucose and gluten (15 g per litre) lysed according to Example 3, and mineral salts to distilled water. The producer of lactic acid used was thermophilic isolate of *Bacillus coagulans.*
The composition of mineral salts was the following:

| | |
|---|---|
| KH₂PO₄ | 0,2 gl⁻¹, |
| MgSO₄·7H₂O | 0,2 gl⁻¹ |
| MnSO₄·4,5H₂O | 10 mgl⁻¹. |

CaCO₃ was used as a neutraliser. 40% of the glucose was used upon fermentation and into lactate with 94% yield, whereas the concentration of the latter reached 5.4% (in relation to lactic acid).
b) In the following fermentation, upon composing of the feed using the same additives to the fractionation water of grains (as described in Example 2) was used. The feed was used as the fermentation medium for producing lactic acid by the *Bacillus coagulans* strain. CaCO₃ was used as a neutraliser. Upon fermentation, 58% of the glucose was utilized, which was transformed in 94% into lactate, and the concentration of which in the medium rose to 7.6% (in relation to lactic acid).
c) Adding 5g of yeast extract per litre of the final fermentation medium resulted in full utilisation of glucose in the fermentation medium, the final lactate concentration of 10.1 % and a yield of 90.2% of the glucose utilized.
d) Full utilisation of glucose and final lactate concentration of 10.1 % was obtained from the substitution of neutralising calcium carbonate with an aqueous solution of 4 M ammonia.

### Example 6

Upon substitution of the yeast extract described in Examples 5c and 5d with a lysate of cells of *Bacillus coagulans* (obtained using proteases Flavourzyme and Neutrase, NovoNordisk, Denmark), 97.2% of the glucose directed to the feed was spent, fermenting 90.2% of sugars into lactate with final concentration of 9.6% (related to the lactic acid).

### Example 7

### Polymerisation of Lactate

The obtained 800 g of 80% aqueous solution of *L*-lactate was heated under continuous stirring and argon flow until the water was separated, after which the catalyst was added. The temperature of the reaction medium was increased up to 180 °C and the pressure in the system was lowered to about 6 millibars. Under optimal temperature and pressure conditions a vitreous mass with very good transparency was produced, which was then cooled down to room temperature in an argon flow. The amount of formed mass was 386 g. When melted, the produced polymer could be drawn into long threads (over 10 metres), or used for coating paper sheets, making them waterproof.

### Use of lactic acid polymer

The obtained pure lactic acid polymer is seldom used in consumer goods due to its brittleness. Its properties can be modified according to the planned application, the characteristics of the environment in the place where the object happens to be used, and where it should be decomposed. Thus, it is necessary to choose the polymer whose properties fulfil its functions as a consumer product and then decompose within the required time in the planned environment.

In order to improve some physical properties of lactic acid polymers, including flexibility, toughness and impact resistance, and for modification of its crystallinity and biodegradability as requested, several additives are used for manufacturing final plastics both upon co-polymerisation with lactic acid (for example, lactic acid with aliphatic and/or aromatic dicarboxylic acid by mediation of oligoalkyleneoxide or lactic acid together with glycol and dicarboxylic acid or lactic acid with polyuretan or aromatic polyester, etc.) as well as for forming physical blends with a lactic acid polymer, for example, blends with other polymers (polyethylenglycol, polypropyleneglycol, polybutylene ether or their blends).

As a thermoplastic polymer, the lactic acid polymer can be used as a material for packing any formed products. One can pack foodstuffs, medical products, items and constructions made from the given polymer. Among other things, it is a suitable material for the base plastic of dipers, because lactic acid polymer is not a favourable growth medium for microbes and thus, it is safer than plastics that are based on cellulose. The lactic acid polymer is easily modifiable into microfibres and can thus be used in textiles which by their qualities such as low binding of humidity, fast drying, no wrinkles, low flammability, good coverage, washability and pleasant weft, can be used to replace natural materials such as silk and cotton. It can also be used for making robes.

The pure, additive-free and unmodified lactic acid polymer is usable in medicine for temporary bandages and supports as well as a self-soluble immobilisation base of locally administrable analgesics, antibiotics and other medical products.

### Lactic acid polymer and environmental protection

The pure, additive-free and unmodified lactic acid polymer is a decomposable material which is decomposed at a temperature of over 57 °C within several days to a couple of weeks, depending on the decomposition technology. The lactic acid polymer decomposes without composting it, but it takes a lot more time.

Modified lactic acid products that are designed for external use and which are to be used at different levels of duration of consumption and biodegradability can be decomposed within a period ranging from a few months up to a couple of years, this goes for in nature as well. Pursuant to one's request, such a plastic of the lactic acid polymer can be designed to be degradable by humidity and/or soil microbes. The plastic can be burnt, because it burns emitting little smoke and without emitting any poisonous gases. The sole burning products are carbon dioxide and water.

## Claims

1. A method for producing the biodegradable lactic acid polymer from cereal starch **characterized in that** the source materials used include cereals such as wheat, rye, triticale, oat, barley or corn, wherein the microbiological synthesis of lactic acid is carried out with thermotolerant bacteria, using components of cereal as sources of mineral and nitrogen compounds in order to produce biodegradable lactic acid polymers in a single integrated production cycle consisting of the following steps:
- producing the starch by fractionation the source material,
- producing the fermentation medium by liquefaction and saccharification of the starch,
- prefermentation and fermentation of the fermentation medium using growth factors,
- purification of the lactate over lactic acid esters or salts,
- synthesis of lactide,
- polymerisation of polylactic acid (PLA).

2. A method according to claim 1 **characterized in that** upon fractionation of cereal into fractions of starch, protein and fibre, the water released upon concentration, esterification and polylactic acid (PLA) synthesis is utilized.

3. A method according to claim 1 or 2 **characterized in that** the water which is released upon fractionation of cereal into fractions of starch, protein and fiber is used for formation of the fermentation medium.

4. A method according to claims 1 to 3 **characterized in that** the water used for fractionation of cereal and partially the protein fraction is utilized for production of lactic acid as the source of N-compounds, vitamins and mineral salts.

5. A method according to any of the preceding claims **characterized in that** the starch, the sugars dissolved in the water utilized for fractionation of cereal and the starch remaining into the protein fraction, are used as sources of the sugar fermented into lactic acid.

6. A method according to any of the preceding claims **characterized in that** the starch contained in the water used for fractionation as well as the starch in the protein fraction, depending on the microorganism used before fermentation, are liquefied or liquefied and saccharinized.

7. A method according to any preceding claims **characterized in that** the saccharification and liquefaction of starch and of the starch dissolved and remaining in fractionation waters occurs in the same reactor.

8. A method according to any preceding claims **characterized in that** the liquefaction and saccharification of the starch remaining in the protein fraction occurs parallel to proteolysis of proteins and is carried out in the reactors of liquefaction and saccharification of starch and it may continue upon fermentation.

9. A method according to claims 1-7 **characterized in that** the liquefaction and saccharification of the starch remaining in the protein fraction occurs parallel to proteolysis of proteins and is carried out in a separate reactor.

10. A method according to any preceding claims **characterized in that** the protein remaining in the fractionation water of grains, the starch fraction and the protein fraction, is subjected to proteolysis upon production of the fermentation medium.

11. A method according to claim 10 **characterized in that** the proteolysis of proteins occurs with a mixture of peptidases and carboxypeptidases, the optimal pH of the proteolysis of which remains between 5 and 7 and the temperature-tolerance of which is up to 55 degrees.

12. A method according to any preceding claims **characterized in that** the bacterial mass generated in the course of fermentation is used as an additive in production of concentrated forage from fiber and/or from the protein fraction or after a preliminary proteolysis upon production of the fermentation medium, as a source of N-compounds and growth factors.

13. A method according to claim 1 **characterized in that** thermotolerant bacteria such as lactobacilli can be replaced by oxygen-tolerant bacilli.

14. A method according to any preceding claims **characterized in that** in the said cycle glucose fermenting strains of lactobacilli and bacilli are used.

15. A method according to claim 14 **characterized in that** in the said cycle glucose fermenting strains of lactobacilli and bacilli having a little auxotrophy and high temperature-tolerance are used.

16. A method according to claim 1 **characterized in that** the separation of lactate and purification from the medium is carried out according to any developed scheme that is adjustable to repetitive utilisation of the water integral production scheme according to claims 2-11.

17. A method according to any preceding claims **characterized in that** cyclical esters of lactate necessary for synthesis of polylactic acid (PLA), are obtained from lactate esters or prepolymer or lactate salts or a combination thereof.

18. A method according to any preceding claims **characterized in that** the thermal energy of water released upon estrification and polymerisation and cooling waters is utilized for liquefaction and saccharification of starch.

## Patentansprüche

1. Verfahren zur Herstellung von biologisch abbaubarem Milchsäure-Polymerisat aus Getreidestärke **dadurch gekennzeichnet, dass** die verwendeten Grundstoffe grundsätzlich aus Getreide bestehen, wie zum Beispiel Weizen, Roggen, Triticale (eine Kreuzung aus Weizen und Roggen, Hafer, Gerste oder Korn, wobei die mikrobiologische Synthese der Milchsäure mit einer thermotoleranten Bakterie herbeigeführt wird, dabei werden Komponenten des Getreides als Quelle für die Mineral- und Stickstoffverbindung benutzt für Herstellung des biologisch abbaubaren Milchsäurepolymeren in einem integrierten Produktionszyklus, der sich aus den folgenden Schritten zusammensetzt:
- Herstellung der Stärke durch Fraktionirung der Ausgangsstoffe
- Herstellung des Fermentierungsmedium durch Verflüssigung und Verzuckerung der Stärke
- Vorfermentierung und Fermentierung des Fermentierungsmedium unter Anwendung des Wachstumsfaktors
- Klärung des Laktats durch Milchsäure-Ester oder -Salze
- Synthese des Laktids
- Herstellung der polymeren Milchsäure (PLA) durch polymerisation

2. Verfahren nach Anspruch 1 **dadurch gekennzeichnet, dass** die Zerlegung des Getreides in Bruchteile von Stärke, Proteine und Fasern erfolgt und dass das Wasser, das durch die Konzentration, Veresterung und polymere Milchsäure (PLA) Synthese freigesetzt wird, verwendet wird.

3. Verfahren nach einem der Anspruch 1 oder 2 **dadurch gekennzeichnet, dass** das Wasser, das durch die Zerlegung des Getreides in Bruchteile von Stärke, Proteine und Fasern freigesetzt wird, für die Bildung des Fermentierungsmedium verwendet wird.

4. Verfahren nach einem der Ansprüche 1 bis 3 **dadurch gekennzeichnet, dass** das Wasser, das für die Zerlegung des Getreides und teilweise für die Proteinspaltung verwendet wird, bei der Herstellung der Milchsäure als die Quelle der N-Komponenten, Vitamine und Mineralsalze zur Anwendung kommt.

5. Verfahren gemäß aller vorangegangenen Ansprüche **dadurch gekennzeichnet, dass** die Stärke, die Verzuckerungen aufgelöst in dem Wasser, das für die Zerlegung des Getreides verwendet wird und die Stärke, die in der Proteinzerlegung zurückgeblieben ist, als Quelle für die Zuckerfermentierung in Milchsäure verwendet wird.

6. Verfahren gemäß aller vorangegangenen Ansprüche **dadurch gekennzeichnet, dass** die in dem Wasser enthaltene Stärke, das für die Zerlegungen angewandt wird, ebenso wie die Stärke in der Proteinzerlegung, abhängig von den Mikroorganismen, die vor der Fermentierung angewendet wurden, verflüssigt werden, oder verflüssigt und verzuckert werden kann.

7. Verfahren gemäß aller vorangegangenen Ansprüchen **dadurch gekennzeichnet, dass** die Verzuckerung und die Verflüssigung der Stärke und die aufgelöste und im Zerlegungswasser zurückgebliebene Stärke in dem gleichen Reaktor erfolgen.

8. Verfahren gemäß aller vorangegangenen Ansprüche **dadurch gekennzeichnet, dass** die Verzuckerung und die Verflüssigung der in der Proteinzerlegung verbliebenen Stärke parallel zu der Proteinspaltung erfolgt und die in den Reaktoren für die Verflüssigung und die Verzuckerung der Stärke vorgenommen wird, und die nach der Fermentierung fortgesetzt werden kann.

9. Verfahren gemäß der Ansprüche 1 bis 7 **dadurch gekennzeichnet, dass** die Verflüssigung und die Verzuckerung der in der Proteinzerlegung verbliebenen Stärke parallel zu der Proteinspaltung erfolgt und in einem getrennten Reaktor durchgeführt wird.

10. Verfahren gemäß aller vorangegangenen Ansprüchen **dadurch gekennzeichnet, dass** das in dem Zerlegungswasser der Körner verbliebene Protein, die Stärkezerlegung und die Proteinzerlegung einer Proteinspaltung für die Herstellung des Fermentationsmittels unterzogen werden.

11. Verfahren nach Anspruch 10 **dadurch gekennzeichnet, dass** die Proteinspaltung stattfindet bei einer Mischung von Peptidasen und Carboxypeptidasen, das optimale pH für die Proteinspaltung hält sich zwischen 5 und 7 und die Temperaturtoleranz beträgt 55 Grad.

12. Verfahren gemäß aller vorangegangenen Ansprüche **dadurch gekennzeichnet, dass** die während der Fermentierung gebildete Bakterienmasse als Additivstoff verwandt wird bei der Herstellung von Futterkonzentraten auf der Basis von Fasern und/oder aus der Proteinspaltung oder nach einer vorbereitenden Proteinspaltung für die Herstellung des Fermentierungsmittels, als Ausgangsstoffe für N-Komponenten und Wachstumsfaktoren.

13. Verfahren nach Anspruch 1 **dadurch gekennzeichnet, dass** die thermotoleranten Bakterien, wie zum Beispiel die Laktobakterien durch die sauerstoff-toleranten Bakterien ersetzt werden können.

14. Verfahren gemäß aller vorangegangenen Ansprüchen **dadurch gekennzeichnet, dass** in den besagten Zyklen alle Glukose-Fermentierungsbakterienstämme der Laktobakterien und Bakterien zur Anwendung kommen.

15. Verfahren nach Anspruch 14 **dadurch gekennzeichnet, dass** in den besagten Zyklen alle Glukose-Fermentierungsbakterienstämme der Laktobakterien und Bakterien zur Anwendung kommen, die über eine leichte Auxotrophie und hohe Temperaturbeständigkeit verfügen.

16. Verfahren nach Anspruch 1 **dadurch gekennzeichnet, dass** die Trennung des Laktats und die Klärung des Medium nach einem entwickelten System durchgeführt wird, die sich der wiederholten Verwendung des Wassers der integrierten Herstellungschemata anpassen, entsprechend den Ansprüchen 2 bis 11.

17. Verfahren gemäß aller vorangegangenen Ansprüche **dadurch gekennzeichnet** die Ableitung von zyklischen Laktat-Esteren aus, die erforderlich sind für die Synthese der polymeren Milchsäure (PLA), gewonnen aus Laktat-Esteren oder Prepolymer oder Laktat-Salzen oder einer Kombination daraus.

18. Verfahren gemäß aller vorangegangenen Ansprüchen **dadurch gekennzeichnet, dass** die thermische Energie des Wassers, ausgelöst durch die Estrifikation und Polymerisierung und das Kühlwasser für die Verflüssigung und Verzuckerung von Stärke zur Anwendung kommt.

## Revendications

1. Un procédé de fabrication des polymères d'acide lactique biodégradables à partir de l'amidon de céréale **caractérisé en ce que** les matériaux sources utilisés incluent des céréales telles que le blé, seigle, triticale, avoine, orge ou maïs, où la synthèse microbiologique de l'acide lactique est réalisée avec des bactéries thermorésistantes, utilisant les composants de céréales comme source de minéraux et composés azotés et afin de produire des polymères d'acide lactique biodégradables en un seul cycle de production intégré comprenant les étapes suivantes:
- production de l'amidon par fractionnement du matériau source,
- production du milieu de fermentation par liquéfaction et saccharification de l'amidon,
- pré-fermentation et fermentation du milieu de fermentation en utilisant des facteurs de croissance,
- purification du lactate des esters ou sels de l'acide lactique,
- synthèse de lactide,
- polymérisation d'acide polylactique (PLA).

2. Procédé selon la revendication 1 **caractérisé en ce que** lors du fractionnement des céréales en fractions d'amidon, protéiques et de fibres, l'eau relâchée lors de la concentration, estérification et synthèse d'acide polylactique (PLA) est utilisée.

3. Procédé selon la revendication 1 ou 2 **caractérisé en ce que** l'eau qui est relâchée lors du fractionnement des céréales en fractions d'amidon, protéiques et de fibres est utilisée pour la formation du milieu de fermentation.

4. Procédé selon les revendications 1 à 3 **caractérisé en ce que** l'eau utilisée pour le fractionnement des céréales et partiellement la fraction protéique sont utilisées pour la production d'acide lactique comme source de composés azotés, vitamines et sels minéraux.

5. Procédé selon l'une quelconque des revendications précédentes **caractérisé en ce que** l'amidon, les sucres dissous dans l'eau utilisée pour le fractionnement des céréales et l'amidon restant dans la fraction protéique, sont utilisés comme sources de sucre fermenté en acide lactique.

6. Procédé selon l'une quelconque des revendications précédentes **caractérisé en ce que** l'amidon contenu dans l'eau utilisée pour le fractionnement ainsi que l'amidon dans la fraction protéique, selon le microorganisme utilisé avant la fermentation, sont liquéfiés ou liquéfiés et saccharinés.

7. Procédé selon l'une quelconque des revendications précédentes **caractérisé en ce que** la saccharification et liquéfaction de l'amidon et de l'amidon dissout et restant dans les eaux de fractionnement a lieu dans le même réacteur.

8. Procédé selon l'une quelconque des revendications précédentes **caractérisé en ce que** la saccharification et liquéfaction de l'amidon restant dans la fraction protéique a lieu en parallèle de la protéolyse des protéines, est effectuée dans les réacteurs de liquéfaction et saccharification de l'amidon et peut continuer lors de la fermentation.

9. Procédé selon les revendications 1 à 7 **caractérisé en ce que** la saccharification et liquéfaction de l'amidon restant dans la fraction protéique a lieu en parallèle de la protéolyse des protéines, est effectuée dans le réacteur séparé.

10. Procédé selon l'une quelconque des revendications précédentes **caractérisé en ce que** les protéines restantes dans l'eau de fractionnement des grains, la fraction d'amidon et la fraction protéique, sont soumises à la protéolyse lors de la production du milieu de fermentation.

11. Procédé selon la revendication 10 **caractérisé en ce que** la protéolyse des protéines se produit avec un mélange de peptidases et carboxypeptidases, le pH optimal de la protéolyse restant entre 5 et 7 et la tolérance de température est jusqu'à 55 degrés.

12. Procédé selon l'une quelconque des revendications précédentes **caractérisé en ce que** la masse bactérienne produite au cours de la fermentation est utilisée comme additif dans la production de fourrage concentré de fibre et/ou de la fraction protéique ou après protéolyse préliminaire lors de la production du milieu de fermentation, comme source de composés azotés et facteurs de croissance.

13. Procédé selon la revendication 1 **caractérisé en ce que** les bactéries thermorésistantes telles que les lactobacilles peuvent être remplacées par des bacilles tolérants à l'oxygène.

14. Procédé selon l'une quelconque des revendications précédentes **caractérisé en ce que** dans ledit cycle sont utilisées toutes les souches de lactobacilles et bacilles fermentant le glucose.

15. Procédé selon la revendication 14 **caractérisé en ce que** dans ledit cycle sont utilisées les souches de lactobacilles et bacilles fermentant le glucose possédant une faible auxotrophie et une tolérance de température élevée.

16. Procédé selon la revendication 1 **caractérisé en ce que** la séparation du lactate et purification du milieu se produit selon n'importe quel procédé développé qui est adaptable à l'utilisation réitérée du système intégral de production d'eau selon les revendications 2 à 11.

17. Procédé selon l'une quelconque des revendications précédentes **caractérisé en ce que** pour dériver les esters cycliques de lactate nécessaires pour la synthèse de l'acide polylactique (PLA), ils proviennent d'esters de lactate ou prépolymères ou sels de lactate ou une combinaison des méthodes susmentionnées.

18. Procédé selon l'une quelconque des revendications précédentes **caractérisé en ce que** l'énergie thermique du relâchement de l'eau lors de l'estérification et la polymérisation et des eaux de refroidissement est utilisée pour la liquéfaction et saccharification de l'amidon.
